# EUROPÄISCHE PATENTSCHRIFT

(11) **EP 3 344 637 B1**
(45) Veröffentlichungstag und Bekanntmachung des Hinweises auf die Patenterteilung: **07.08.2019**
(21) Anmeldenummer: 16757896.2
(22) Anmeldetag: 30.08.2016
(51) Int. Cl.: C07F 9/30, C07F 9/32

(54) **VERFAHREN ZUR HERSTELLUNG VON PHOSPHORHALTIGEN CYANHYDRINESTERN**
METHOD FOR PRODUCING CYANOHYDRIN ESTERS CONTAINING PHOSPHOROUS
PROCÉDÉ DE PRÉPARATION D'ESTERS DE CYANHYDRINE CONTENANT DU PHOSPHORE

(30) Priorität: 02.09.2015 EP 15183422
(43) Veröffentlichungstag der Anmeldung: 11.07.2018
(73) Patentinhaber: BASF SE, 67056 Ludwigshafen (DE)
(72) Erfinder: RESSEL, Hans-Joachim, 65795 Hattersheim (DE); TELLMANN, Kilian, 50823 Köln (DE); FORD, Mark James, 65207 Wiesbaden-Breckenheim (DE); LITTMANN, Martin, 51375 Leverkusen (DE); SCHLEGEL, Günter, 51381 Leverkusen (DE)
(74) Vertreter: BASF IP Association
(86) Internationale Anmeldenummer: PCT/EP2016/070349
(87) Internationale Veröffentlichungsnummer: WO 2017/037034

(56) Entgegenhaltungen:
- US-A- 4 521 348

## Beschreibung

Die vorliegende Erfindung betrifft primär ein Verfahren zur Herstellung bestimmter phosphorhaltiger Cyanhydrinester der nachfolgend definierten Formel (I) und deren Verwendung zur Herstellung von Glufosinat bzw. von Glufosinat-Salzen. Ferner betrifft die vorliegende Erfindung ein Verfahren zur Herstellung von Glufosinat bzw. von Glufosinat-Salzen.

Phosphorhaltige Cyanhydrinester sind wertvolle Zwischenprodukte auf verschiedenen technischen Gebieten, insbesondere zur Herstellung biologisch aktiver Substanzen, die im pharmazeutischen bzw. agrochemischen Bereich eingesetzt werden können.

US 4,168,963 beschreibt diverse phosphorhaltige herbizid wirksame Verbindungen, von denen insbesondere Phosphinothricin (2-Amino-4-[hydroxy(methyl)phosphinoyl]-butansäure; Kurzbezeichnung ("common name"): Glufosinate, nachfolgend Glufosinat) bzw. dessen Salze kommerzielle Bedeutung im Bereich der Agrochemie (Agrarchemie) erlangt haben.

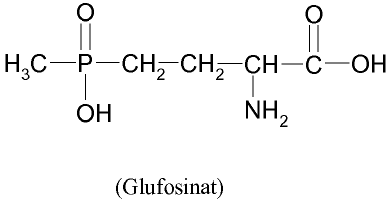

Methoden zur Herstellung von Zwischenprodukten zur Synthese solcher phosphorhaltiger herbizid wirksamer Verbindungen, insbesondere von Glufosinat, sind beispielsweise in US 4,521,348, US 4,599,207 und US 6,359,162B1 beschrieben.

Umsetzungen von Cyanhydrinestern und Methanphosphonigsäureestern sind beispielsweise in US 4,521,348 oder US 4,599,207 beschrieben.

Die Verfahren aus dem Stand der Technik zur Herstellung phosphorhaltiger Cyanhydrinester erlauben zwar die Herstellung der gewünschten phosphorhaltigen Cyanhydrinester in teils sehr guter Ausbeute, weisen jedoch noch Nachteile auf, wie beispielsweise noch verbesserbare Ausbeuten an phosphorhaltigen Cyanhydrinestern, einen zu hohen Anteil an Koppel- oder Nebenprodukten, einen zu hohen Aufwand bei der Reinigung bzw. Isolierung der phosphorhaltigen Cyanhydrinester und/oder zu schwierige verfahrens-bzw. anlagentechnisch Reaktionsbedingungen.

Aufgabe der vorliegenden Erfindung war es daher ein Verfahren zur Herstellung phosphorhaltiger Cyanhydrinester zu finden, welches die phosphorhaltigen Cyanhydrinester in weiter verbesserter Ausbeute liefert und/oder einen geringeren Anteil an Koppel- oder Nebenprodukten ergibt, und zudem dabei vorzugsweise eine verbesserte Reaktionsführung ermöglicht, beispielsweise in Bezug auf sicherheits-, umweit- und/oder qualitätsrelevante Aspekte.

Das nachfolgend beschriebene erfindungsgemäße Verfahren erfüllt diese Aufgabe.

Gegenstand der vorliegenden Erfindung ist ein Verfahren zur Herstellung phosphorhaltiger Cyanhydrinester der Formel (I) 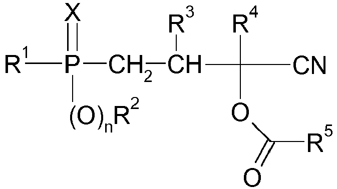 dadurch gekennzeichnet, dass eine Verbindung der Formel (II) 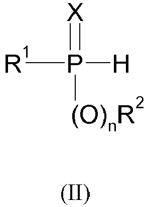 mit einer Verbindung der Formel (III) 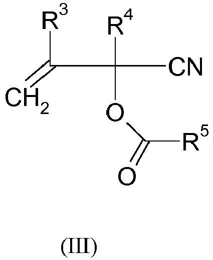 wobei jeweils gilt:
- R¹: bedeutet (C₁-C₁₂)-Alkyl, (C₁-C₁₂)-Haloalkyl, (C₆-C₁₀)-Aryl, (C₆-C₁₀)-Haloaryl, (C₇-C₁₀)-Aralkyl, (C₇-C₁₀)-Haloaralkyl, (C₄-C₁₀)-Cycloalkyl oder (C₄-C₁₀)-Halocycloalkyl,
- R²: bedeutet (C₁-C₁₂)-Alkyl, (C₁-C₁₂)-Haloalkyl, (C₆-C₁₀)-Aryl, (C₆-C₁₀)-Haloaryl, (C₇-C₁₀)-Aralkyl, (C₇-C₁₀)-Haloaralkyl, (C₄-C₁₀)-Cycloalkyl oder (C₄-C₁₀)-Halocycloalkyl,
- R³ und R⁴: bedeuten jeweils unabhängig voneinander Wasserstoff, (C₁-C₄)-Alkyl, Phenyl oder Benzyl,
- R⁵: bedeutet (C₁-C₁₂)-Alkyl, (C₁-C₁₂)-Haloalkyl, (C₆-C₁₀)-Aryl, (C₆-C₁₀)-Haloaryl, (C₇-C₁₀)-Aralkyl, (C₇-C₁₀)-Haloaralkyl, (C₄-C₁₀)-Cycloalkyl oder (C₄-C₁₀)-Halocycloalkyl,
- X: bedeutet Sauerstoff oder Schwefel, und
- n: ist 0 oder 1,
in Gegenwart einer oder mehrerer radikalbildender Substanzen (IV) umgesetzt wird, wobei

ein Dosierstrom (D1) in den Reaktor dosiert wird, welcher eine oder mehrere Verbindungen der Formel (II) und eine oder mehrere radikalbildende Substanzen (IV) enthält, und wobei Dosierstrom (D1) 25 - 100 mol.-% der Gesamtmenge der in die Umsetzung insgesamt eingesetzten Menge der radikalbildenden Substanzen (IV) enthält.

Mit dem erfindungsgemäßen Verfahren, insbesondere in einer der als bevorzugt bzw. als besonders bevorzugt bezeichneten Ausgestaltungen des erfindungsgemäßen Verfahrens, werden die phosphorhaltigen Cyanhydrinester der Formel (I) bzw. den weiter unten definierten Formeln (Ia) bzw. (Ib) in besserer Ausbeute und regelmäßig in höherer Reinheit erhalten.

Insgesamt entstehen in den erfindungsgemäßen Verfahren, auch in dem weiteren nachfolgend beschriebenen erfindungsgemäßen Verfahren zur Herstellung von Glufosinat, weniger unerwünschte Nebenkomponenten, so dass die erfindungsgemäßen Verfahren effizienter und energiesparender sind.

Die jeweiligen Alkylreste der Reste R¹, R², R³, R⁴ und R⁵ können im Kohlenstoffgerüst jeweils geradkettig oder verzweigtkettig (verzweigt) sein.

Der Ausdruck "(C₁-C₄)-Alkyl" ist dabei die Kurzschreibweise für einen Alkylrest mit 1 bis 4 Kohlenstoffatomen, d.h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z.B. "(C₁-C₆)-Alkyl" umfassen entsprechend auch gradkettige oder verzweigte Alkylreste mit einer größeren Zahl von C-Atomen, d.h. gemäß Beispiel auch die Alkylreste mit 5 und 6 C-Atomen.

"Halogen" bezieht vorzugsweise auf die Gruppe bestehend aus Fluor, Chlor, Brom und Iod. Haloalkyl, Haloaryl, Haloaralkyl und Halocycloalkyl bedeuten durch gleiche oder verschiedene Halogenatome, vorzugsweise aus der Gruppe Fluor, Chlor und Brom, insbesondere aus der Gruppe Fluor und Chlor, teilweise oder vollständig substituiertes Alkyl, Aryl, Aralkyl bzw. Cycloalkyl. So umfasst Haloalkyl z.B. Monohaloalkyl (= Monohalogenalkyl), Dihaloalkyl (= Dihalogenalkyl), Trihaloalkyl (= Trihalogenalkyl), oder auch Perhaloalkyl, wie beispielsweise CF₃, CHF₂, CH₂F, CF₃CF₂, CH₂FCHCl, CCl₃, CHCl₂, CH₂CH₂Cl. Entsprechendes gilt für die anderen durch Halogen substituierte Reste.

Als Verbindungen der Formel (II) sind unter anderem die folgenden geeignet und bevorzugt: Methanphosphonigsäuremono(C₁-C₆)-Alkylester, Methanphosphonigsäuremono-Dodecylester, Methanphosphonigsäuremono-Phenylester; Ethanphosphonigsäuremono(C₁-C₆)-Alkylester, Ethanphosphonigsäuremono-Dodecylester, Ethanphosphonigsäuremono-Phenylester; Propanphosphonigsäuremono(C₁-C₆)-Alkylester, Propanphosphonigsäuremono-Dodecylester, Propanphosphonigsäuremono-Phenylester; Butanphosphonigsäuremono(C₁-C₆)-Alkylester, Butanphosphonigsäuremono-Dodecylester, Butanphosphonigsäuremono-Phenylester; Phenylphosphonigsäuremono(C₁-C₆)-Alkylester, Phenylphosphonigsäuremono-Dodecylester, Phenylphosphonigsäuremono-Phenylester; Benzylphosphonigsäuremono(C₁-C₆)-Alkylester, Benzylphosphonigsäuremono-Dodecylester, Benzylphosphonigsäuremono-Phenylester; Methylthiophosphonigsäuremono(C₁-C₆)-Alkylester, Methylthiophosphonigsäuremono-Dodecylester Methylthiophosphonigsäuremono-Phenylester; Dimethylphosphinoxid, Diethylphosphinoxid, Dipropylphosphinoxid, Dibutylphosphinoxid, Diphenylphosphinoxid, Methyl-Phenylphosphinoxid, Dibenzylphosphinoxid, Dimethylphosphinsulfid, und Diphenylphosphinsulfid.

Die Herstellung der Verbindungen der Formel (II) ist dem Fachmann bekannt und kann nach literaturbekannten Verfahren erfolgen (z.B. US 3,914,345; US 4,474,711; US 4,485,052; US 4,839,105; US 5,128,495).

Die Herstellung der Cyanhydrinester der Formel (III) ist dem Fachmann ebenfalls bekannt und kann nach literaturbekannten Verfahren erfolgen (z.B. gemäß EP 0 019 750 A1 bzw. gemäß US 4,521,348 und den darin zitierten entsprechenden Dokumenten).

Vorzugsweise gilt für das erfindungsgemäße Verfahren:
R³ und R⁴ bedeuten jeweils unabhängig voneinander Wasserstoff oder Methyl,
und/oder
X bedeutet Sauerstoff,
und/oder
n ist 1.

Das erfindungsgemäße Verfahren betrifft vorzugsweise die Herstellung phosphorhaltiger Cyanhydrinester der Formel (Ia) 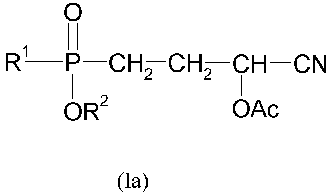 dadurch gekennzeichnet, dass eine Verbindung der Formel (IIa) 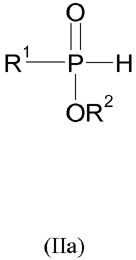 mit einem Acroleincyanhydrinester der Formel (IIIa) umgesetzt wird, wobei eine der Verbindungen oder die Verbindung der Formel (III) der Formel (IIIa) entspricht (Acroleincyanhydrin-O-acetat, Ac = Acetyl, entsprechend bedeutet R⁵ in Formel (I) = Methyl) 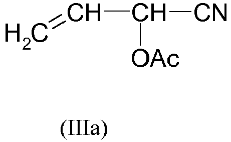 wobei jeweils gilt:
- R¹: (C₁-C₆)-Alkyl, (C₁-C₆)-Haloalkyl, (C₆-C₈)-Aryl, (C₆-C₈)-Haloaryl, (C₇-C₁₀)-Aralkyl, (C₇-C₁₀)-Haloaralkyl, (C₅-C₈)-Cycloalkyl oder (C₅-C₈)-Halocycloalkyl bedeutet, und
- R²: (C₁-C₆)-Alkyl, (C₁-C₆)-Haloalkyl, (C₆-C₈)-Aryl, (C₆-C₈)-Haloaryl, (C₇-C₁₀)-Aralkyl, (C₇-C₁₀)-Haloaralkyl, (C₅-C₈)-Cycloalkyl oder (C₅-C₈)-Halocycloalkyl bedeutet.

Bevorzugt gilt dabei jeweils:
- R¹: bedeutet (C₁-C₄)-Alkyl oder (C₁-C₄)-Haloalkyl, bevorzugt Methyl oder Ethyl,
- R²: bedeutet (C₁-C₆)-Alkyl oder (C₁-C₆)-Haloalkyl, bevorzugt (C₃-C₆)-Alkyl, dabei wiederum bevorzugt C₄-Alkyl oder C₅-Alkyl.

In dem erfindungsgemäßen Verfahren bedeutet in Formel (I) bzw. in Formel (Ia)
R¹ besonders bevorzugt Methyl, und
R² besonders bevorzugt (C₁-C₆)-Alkyl, dabei wiederum bevorzugt (C₄-C₅)-Alkyl.

Die nachfolgenden Ausführungen und die als bevorzugt bzw. besonders bevorzugt gekennzeichneten Ausgestaltungen des erfindungsgemäßen Verfahrens gelten insbesondere für die Umsetzung einer Verbindung der Formel (IIa), in der R¹ Methyl bedeutet (und damit der nachfolgend definierten Verbindung der Formel (IIb) entspricht) und R² (C₁-C₆)-Alkyl bedeutet, mit dem Acroleincyanhydrinester der Formel (IIIa).

In dem erfindungsgemäßen Verfahren wird zumindest eine Teilmenge der insgesamt eingesetzten Gesamtmenge der radikalbildenden Substanzen (IV) mit zumindest einer Teilmenge der insgesamt eingesetzten Gesamtmenge der Verbindungen der Formel (II) bzw. (IIa) vermischt, bevor der resultierende Dosierstrom (D1) in den Reaktor dosiert wird.

Das erfindungsgemäße Verfahren ist vorzugsweise dadurch gekennzeichnet, dass zwei separate Dosierströme (D1) und (D2) in den Reaktor dosiert werden, und diese Dosierströme (D1) und (D2) folgende Zusammensetzung aufweisen:
Dosierstrom (D1) enthält eine oder mehrere Verbindungen der Formel (II) und eine oder mehrere radikalbildende Substanzen (IV), wobei Dosierstrom (D1) 25 - 100 mol.-% der Gesamtmenge der in die Umsetzung insgesamt eingesetzten Menge der radikalbildenden Substanzen (IV) enthält,
und
Dosierstrom (D2) enthält eine oder mehrere Verbindungen der Formel (III), sowie gegebenenfalls eine oder mehrere Verbindungen der Formel (II), und gegebenenfalls eine oder mehrere radikalbildende Substanzen (IV).

In dem erfindungsgemäßen Verfahren wird ferner vorzugsweise zumindest eine Teilmenge der insgesamt eingesetzten Gesamtmenge der Verbindungen der Formel (II) bzw. (IIa) mit der oder den Verbindungen der Formel (III) bzw. (IIIa), sowie optional zusätzlich mit einer oder mehrerer radikalbildender Substanzen (IV), vermischt, bevor der resultierende Dosierstrom (D2) in den Reaktor dosiert wird.

In dem erfindungsgemäßen Verfahren werden die im Rahmen der vorliegenden Erfindung definierten Dosierströme (D1) und (D2) aus separaten (d.h. räumlich getrennten) Behältnissen in den Reaktor (d.h. das Reaktionsgefäß) eindosiert.

Ein bevorzugtes erfindungsgemäßes Verfahren ist dadurch gekennzeichnet, dass
Dosierstrom (D1) 30 - 100 mol.-% der Gesamtmenge der in die Umsetzung insgesamt eingesetzten Menge der radikalbildenden Substanzen (IV) enthält, vorzugsweise 40 - 100 mol.-%,bevorzugt 50 - 100 mol.-%, weiter bevorzugt 60 - 100 mol.-%, noch weiter bevorzugt 70 - 100 mol.-%, besonders bevorzugt 80 - 100 mol.-%, insbesondere bevorzugt 90 - 100 mol.-% und am meisten bevorzugt 95 - 100 mol.-%.

Ein weiter bevorzugtes erfindungsgemäßes Verfahren ist dadurch gekennzeichnet, dass
Dosierstrom (D1) 90 - 100 Gew.-%, bevorzugt Dosierstrom (D1) 95 - 100 Gew.-%, weiter bevorzugt 100 Gew.-% der Gesamtmenge der in den Dosierströmen (D1) und (D2) insgesamt eingesetzten Menge an Verbindungen der Formel (II) enthält.

Eine alternative bevorzugte Ausführungsform des erfindungsgemäßen Verfahrens ist dadurch gekennzeichnet, dass Dosierstrom (D1) 10 - 90 Gew.-%, bevorzugt 20 - 80 Gew.-%, weiter bevorzugt 25 - 75 Gew.-%, besonders bevorzugt 30 - 70 Gew.-%, und insbesondere bevorzugt 40 - 60 Gew.-% der Gesamtmenge der in den Dosierströmen (D1) und (D2) insgesamt eingesetzten Menge an Verbindungen der Formel (II) enthält.

Ein bevorzugtes erfindungsgemäßes Verfahren ist dadurch gekennzeichnet, dass
Dosierstrom (D2) 80 - 100 Gew.-%, bevorzugt 90 - 100 Gew.-%, bevorzugt 95 - 100 Gew.-%, insbesondere bevorzugt 100 Gew.-% der Gesamtmenge der in den Dosierströmen (D1) und (D2) insgesamt eingesetzten Menge Verbindungen der Formel (III) enthält.

Ein besonders bevorzugtes erfindungsgemäßes Verfahren ist dadurch gekennzeichnet, dass
Dosierstrom (D1) 40 - 100 mol.-%, vorzugsweise 50 - 100 mol.-%, bevorzugt 60 - 100 mol.-%, weiter bevorzugt 70 - 100 mol.-%, insbesondere bevorzugt 80 - 100 mol.-%, und besonders bevorzugt 90 - 100 mol.-% der Gesamtmenge der in den Dosierströmen (D1) und (D2) insgesamt eingesetzten Menge der radikalbildenden Substanzen (IV) enthält,
und/oder
Dosierstrom (D2) 0 - 60 mol.-%, vorzugsweise 0 - 50 mol.-%, bevorzugt 0 - 40 mol.-%, weiter bevorzugt 0 - 30 mol.-%, insbesondere bevorzugt 0 - 20 mol.-%, und besonders bevorzugt 0 - 10 mol.-% der Gesamtmenge der in den Dosierströmen (D1) und (D2) insgesamt eingesetzten Menge der radikalbildenden Substanzen (IV) enthält.

In einer besonders bevorzugten Ausführungsform ist das erfindungsgemäße Verfahren dadurch gekennzeichnet, dass
Dosierstrom (D1) 90 - 100 mol.-%, vorzugsweise 95 - 100 mol.-%, bevorzugt 97 - 100 mol.-%, weiter bevorzugt 98 - 100 mol.-% der Gesamtmenge der in den Dosierströmen (D1) und (D2) insgesamt eingesetzten Menge der radikalbildenden Substanzen (IV) enthält,
und
Dosierstrom (D2) 0 - 10 mol.-%, vorzugsweise 0 - 5 mol.-%, bevorzugt 0 - 3 mol.-%, weiter bevorzugt 0 - 2 mol.-% der Gesamtmenge der in den Dosierströmen (D1) und (D2) insgesamt eingesetzten Menge der radikalbildenden Substanzen (IV) enthält.

In einer besonders bevorzugten Ausführungsform ist das erfindungsgemäße Verfahren dadurch gekennzeichnet, dass
Dosierstrom (D1) 99 - 100 mol.-%, vorzugsweise 100 mol.-% der Gesamtmenge der in den Dosierströmen (D1) und (D2) insgesamt eingesetzten Menge der radikalbildenden Substanzen (IV) enthält,
und
Dosierstrom (D2) 0 - 1 mol.-%, vorzugsweise 0 mol.-% der Gesamtmenge der in den Dosierströmen (D1) und (D2) insgesamt eingesetzten Menge der radikalbildenden Substanzen (IV) enthält.

Ein besonders bevorzugtes erfindungsgemäßes Verfahren ist dadurch gekennzeichnet, dass die Gesamtmenge der Verbindungen (II) und (IV) im Dosierstrom (D1) 75 bis 100 Gew.-% beträgt, bevorzugt 80 bis 100 Gew.-%, weiter bevorzugt 85 bis 100 Gew.-%, besonders bevorzugt 90 bis 100 Gew.-%, jeweils bezogen auf das Gesamtgewicht des Dosierstroms (D1).

Das erfindungsgemäße Verfahren wird vorzugsweise unter Bedingungen durchgeführt, in denen Radikale gebildet werden.

Die Umsetzung der Verbindungen der Formel (II) und (III) bzw. der Formel (IIa) und (IIIa) zu den Verbindungen der Formel (I) bzw. (Ia) in einem erfindungsgemäßen Verfahren erfolgt vorzugsweise mit Hilfe einer radikalbildende Quelle, beispielsweise unter Einsatz elektromagnetischer Felder wie UV-, Gamma- oder Röntgenstrahlen, elektrischer Felder oder elektrochemischer Methoden, oder in Gegenwart einer oder mehrerer radikalbildender Substanzen.

Im Rahmen des erfindungsgemäßen Verfahrens werden vorzugsweise radikalbildende Substanzen eingesetzt.

Ein bevorzugtes erfindungsgemäßes Verfahren ist dadurch gekennzeichnet, dass eine, mehrere oder sämtliche radikalbildenden Substanzen (IV) der Formel (V) entsprechen 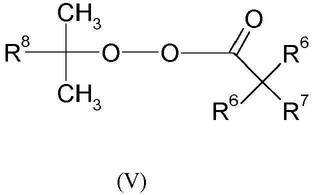 wobei
- R⁶: unabhängig voneinander Wasserstoff, (C₁-C₁₀)-Alkyl bedeutet, vorzugsweise (C₁-C₆)-Alkyl, bevorzugt (C₁-C₄)-Alkyl,
- R⁷: Wasserstoff oder (C₁-C₁₀)-Alkyl bedeutet, vorzugsweise Wasserstoff oder (C₁-C₆)-Alkyl, bevorzugt Wasserstoff oder (C₁-C₄)-Alkyl,
und
- R⁸: Methyl, Ethyl, 2,2-Dimethylpropyl oder Phenyl bedeutet.

Bevorzugt radikalbildenden Substanzen der Formel (V) sind solche, in denen
R⁶ unabhängig voneinander (C₁-C₁₀)-Alkyl bedeutet, vorzugsweise (C₁-C₆)-Alkyl, bevorzugt (C₁-C₄)-Alkyl,
R⁷ Wasserstoff oder (C₁-C₁₀)-Alkyl bedeutet, vorzugsweise Wasserstoff oder (C₁-C₆)-Alkyl, bevorzugt Wasserstoff oder (C₁-C₄)-Alkyl,
und
R⁸ Methyl, Ethyl, 2,2-Dimethylpropyl oder Phenyl bedeutet.

Die Radikalbildner (Radikalstarter) der Formel (V) sind an sich bekannt und zum Teil kommerziell erhältlich.

Die Radikalbildner der Formel (V) sind dabei vorzugsweise gewählt aus der Gruppe bestehend aus tert-Butylperoxypivalat, tert-Amylperoxypivalat, tert-Butylperoxyneodecanoat, 1,1,3,3-Tetramethylbutylperoxyneodecanoat, tert-Butylperoxy-2-ethylhexanoat, 1,1,3,3-Tetramethylbutylperoxy-2-ethylhexanoat, tert-Amylperoxyneodecanoat, Cumylperoxyneodecanoat, Cumylperoxyneoheptanoat, Cumylperoxypivalat, und deren Mischungen.

Die Radikalbildner der Formel (V) sind dabei bevorzugt gewählt aus der Gruppe bestehend aus tert-Butylperoxyneodecanoat, 1,1,3,3-Tetramethylbutylperoxyneodecanoat, tert-Butylperoxy-2-ethylhexanoat, 1,1,3,3-Tetramethylbutylperoxy-2-ethylhexanoat, Cumylperoxyneodecanoat, und deren Mischungen, dabei wiederum besonders bevorzugt sind 1,1,3,3-Tetramethylbutylperoxyneodecanoat, tert-Butylperoxyneodecanoat und/oder tert-Butylperoxy-2-ethylhexanoat.

Das erfindungsgemäße Verfahren ermöglicht die Herstellung der phosphorhaltigen Cyanhydrinester der Formel (I) oder (Ia) bzw. der weiter unten definierten Formel (Ib) unter milden Reaktionsbedingungen und einfacherer verfahrens- bzw. anlagentechnischer Durchführbarkeit. Dadurch können die phosphorhaltigen Cyanhydrinester der Formel (I), (Ia) bzw. (Ib) prozesstechnisch leichter, in noch besseren Ausbeuten und in hoher Reinheit erhalten werden.

Das erfindungsgemäße Verfahren wird vorzugsweise in der Weise durchgeführt, dass die Dosierströme (D1) und (D2) überwiegend simultan, vorzugsweise simultan in den Reaktor dosiert werden.

Das erfindungsgemäße Verfahren wird vorzugsweise in der Weise durchgeführt, dass die Umsetzung bei einer Temperatur im Bereich von 40 bis 120 °C erfolgt, bevorzugt bei einer Temperatur im Bereich von 50 bis 110 °C, weiter bevorzugt bei einer Temperatur im Bereich von 55 bis 100 °C, und besonders bevorzugt bei einer Temperatur im Bereich von 60 bis 95 °C.

So wird bei der Durchführung des erfindungsgemäßen Verfahrens beispielsweise eine Disproportionierung von Edukten der Formel (II) bzw. (IIa) signifikant reduziert oder weitgehend vermieden. Zudem wird bei der Durchführung des erfindungsgemäßen Verfahrens die Oligo- und Polymerisation der Verbindungen der Formel (III) bzw. (IIIa) signifikant reduziert oder weitgehend vermieden.

Es ist im Rahmen des erfindungsgemäßen Verfahrens vorteilhaft, die Cyanhydrinester der Formel (III) bzw. (IIIa) in möglichst hoher Reinheit einzusetzen. Vorzugsweise werden die Cyanhydrinester der Formel (III) bzw. (IIIa) in einer Reinheit von größer oder gleich 90 Gew.-% eingesetzt, bevorzugt von größer oder gleich 92 Gew.-%, weiter bevorzugt von größer oder gleich 95 Gew.-%, insbesondere bevorzugt von größer oder gleich 98 Gew.-%.

Die gebildeten phosphorhaltigen Cyanhydrinester der Formel (I) bzw. (Ia) bzw. der nachfolgend definierten Formel (Ib) können als Ausgangsstoffe zur Synthese von phosphorhaltigen Aminosäuren wie beispielsweise Glufosinat verwendet werden (ein solcher Syntheseweg wird weiter unten näher beschrieben).

Zur Vermeidung unerwünschter Nebenreaktionen und damit zur Erzielung hoher Ausbeuten ist es zudem vorteilhaft, das phosphorhaltige Edukt (II) bzw. (IIa), bezogen auf den Cyanhydrinester der Formel (III) bzw. (IIIa), im molaren Überschuss einzusetzen.

Ferner ist das erfindungsgemäße Verfahren in diesen bevorzugten Ausgestaltungen dahingehend von Vorteil, dass keine hohen Überschüsse von Verbindungen der Formel (II) bzw. (IIa) in Bezug auf die eingesetzte Gesamtmenge Verbindungen der Formel (III) bzw. (IIIa) erforderlich sind, um die vorteilhaften Effekte des erfindungsgemäßen Verfahrens zu erreichen.

Vorzugsweise liegt in dem erfindungsgemäßen Verfahren das molare Verhältnis der Gesamtmenge der eingesetzten Verbindung der Formel (II) bzw. (IIa) zu der Gesamtmenge der eingesetzten Verbindung der Formel (III) bzw. (IIIa) im Bereich von 8 : 1 bis 1 : 1 liegt, vorzugsweise im Bereich von 5 : 1 bis 2 : 1.

Bevorzugt liegt in dem erfindungsgemäßen Verfahren das molare Verhältnis der Gesamtmenge der eingesetzten Verbindung der Formel (II) bzw. (IIa) zu der Gesamtmenge der eingesetzten Verbindung der Formel (III) bzw. (IIIa) im Bereich von 5 : 1 bis 5 : 2 liegt, weiter bevorzugt im Bereich von 9 : 2 bis 5 : 2.

Besonders bevorzugte Ausgestaltungen des erfindungsgemäßen Verfahrens zur Herstellung der Verbindungen der Formel (I) durch Umsetzung einer Verbindung der Formel (II) mit dem Acroleincyanhydrinester der Formel (III), sind dadurch gekennzeichnet, dass
Dosierstrom (D1) 30 - 100 mol.-% der Gesamtmenge der in die Umsetzung insgesamt eingesetzten Menge der radikalbildenden Substanzen (IV) enthält, vorzugsweise 40 - 100 mol.-%,bevorzugt 50 - 100 mol.-%, weiter bevorzugt 60 - 100 mol.-%, noch weiter bevorzugt 70 - 100 mol.-%, besonders bevorzugt 80 - 100 mol.-%, insbesondere bevorzugt 90 - 100 mol.-% und am meisten bevorzugt 95 - 100 mol.-%,
die Gesamtmenge der Verbindungen (II) und (IV) im Dosierstrom (D1) 75 bis 100 Gew.-% beträgt, bevorzugt 80 bis 100 Gew.-%, weiter bevorzugt 85 bis 100 Gew.-%, besonders bevorzugt 90 bis 100 Gew.-% jeweils bezogen auf das Gesamtgewicht des Dosierstroms (D1),
das molare Verhältnis der Gesamtmenge der eingesetzten Verbindung der Formel (II) zu der Gesamtmenge der eingesetzten Verbindung der Formel (III) im Bereich von 8 : 1 bis 1 : 1 liegt, vorzugsweise im Bereich von 5 : 1 bis 2 : 1,
und
die Umsetzung bei einer Temperatur im Bereich von 40 bis 120 °C erfolgt, bevorzugt bei einer Temperatur im Bereich von 50 bis 110 °C, weiter bevorzugt bei einer Temperatur im Bereich von 55 bis 100 °C, und besonders bevorzugt bei einer Temperatur im Bereich von 60 bis 95 °C.

Besonders bevorzugte Ausgestaltungen des erfindungsgemäßen Verfahrens zur Herstellung der Verbindungen der Formel (I) durch Umsetzung einer Verbindung der Formel (II) mit dem Acroleincyanhydrinester der Formel (III), sind dadurch gekennzeichnet, dass
Dosierstrom (D1) 40 - 100 mol.-%, vorzugsweise 50 - 100 mol.-%, bevorzugt 60 - 100 mol.-%, weiter bevorzugt 70 - 100 mol.-%, insbesondere bevorzugt 80 - 100 mol.-%, und besonders bevorzugt 90 - 100 mol.-% der Gesamtmenge der in den Dosierströmen (D1) und (D2) insgesamt eingesetzten Menge der radikalbildenden Substanzen (IV) enthält,
Dosierstrom (D2) 0 - 60 mol.-%, vorzugsweise 0 - 50 mol.-%, bevorzugt 0 - 40 mol.-%, weiter bevorzugt 0 - 30 mol.-%, insbesondere bevorzugt 0 - 20 mol.-%, und besonders bevorzugt 0 - 10 mol.-% der Gesamtmenge der in den Dosierströmen (D1) und (D2) insgesamt eingesetzten Menge der radikalbildenden Substanzen (IV) enthält,
die Gesamtmenge der Verbindungen (II) und (IV) im Dosierstrom (D1) 75 bis 100 Gew.-% beträgt, bevorzugt 80 bis 100 Gew.-%, weiter bevorzugt 85 bis 100 Gew.-%, besonders bevorzugt 90 bis 100 Gew.-% jeweils bezogen auf das Gesamtgewicht des Dosierstroms (D1),
das molare Verhältnis der Gesamtmenge der eingesetzten Verbindung der Formel (II) zu der Gesamtmenge der eingesetzten Verbindung der Formel (III) im Bereich von 8 : 1 bis 1 : 1 liegt, vorzugsweise im Bereich von 5 : 1 bis 2 : 1,
wobei eine, mehrere oder sämtliche radikalbildenden Substanzen (IV) der Formel (V) entsprechen und vorzugsweise gewählt sind aus der Gruppe bestehend aus tert-Butylperoxypivalat, tert-Amylperoxypivalat, tert-Butylperoxyneodecanoat, 1,1,3,3-Tetramethylbutylperoxyneodecanoat, tert-Butylperoxy-2-ethylhexanoat, 1,1,3,3-Tetramethylbutylperoxy-2-ethylhexanoat, tert-Amylperoxyneodecanoat, Cumylperoxyneodecanoat, Cumylperoxyneoheptanoat, Cumylperoxypivalat, und deren Mischungen,
und
die Umsetzung bei einer Temperatur im Bereich von 40 bis 120 °C erfolgt, bevorzugt bei einer Temperatur im Bereich von 50 bis 110 °C, weiter bevorzugt bei einer Temperatur im Bereich von 55 bis 100 °C, und besonders bevorzugt bei einer Temperatur im Bereich von 60 bis 95 °C.

Besonders bevorzugte Ausgestaltungen des erfindungsgemäßen Verfahrens zur Herstellung der Verbindungen der Formel (I) durch Umsetzung einer Verbindung der Formel (II) mit dem Acroleincyanhydrinester der Formel (III), insbesondere zur Herstellung der Verbindungen der Formel (Ia) durch Umsetzung einer Verbindung der Formel (IIa) mit dem Acroleincyanhydrinester der Formel (IIIa), sind dadurch gekennzeichnet, dass
Dosierstrom (D1) 50 - 100 mol.-%, bevorzugt 60 - 100 mol.-%, weiter bevorzugt 70 - 100 mol.-%, insbesondere bevorzugt 80 - 100 mol.-%, und besonders bevorzugt 90 - 100 mol.-% der Gesamtmenge der in den Dosierströmen (D1) und (D2) insgesamt eingesetzten Menge der radikalbildenden Substanzen (IV) enthält,
Dosierstrom (D2) 0 - 50 mol.-%, bevorzugt 0 - 40 mol.-%, weiter bevorzugt 0 - 30 mol.-%, insbesondere bevorzugt 0 - 20 mol.-%, und besonders bevorzugt 0 - 10 mol.-% der Gesamtmenge der in den Dosierströmen (D1) und (D2) insgesamt eingesetzten Menge der radikalbildenden Substanzen (IV) enthält,
die Gesamtmenge der Verbindungen (II) bzw. (IIa) und (IV) im Dosierstrom (D1) 80 bis 100 Gew.-% beträgt, bevorzugt 85 bis 100 Gew.-%, besonders bevorzugt 90 bis 100 Gew.-%, jeweils bezogen auf das Gesamtgewicht des Dosierstroms (D1),
das molare Verhältnis der Gesamtmenge der eingesetzten Verbindung der Formel (II) bzw. (IIa) zu der Gesamtmenge der eingesetzten Verbindung der Formel (III) bzw. (IIIa) im Bereich von 5 : 1 bis 2 : 1 liegt,
wobei eine, mehrere oder sämtliche radikalbildenden Substanzen (IV) der Formel (V) entsprechen und vorzugsweise gewählt sind aus der Gruppe bestehend aus tert-Butylperoxypivalat, tert-Amylperoxypivalat, tert-Butylperoxyneodecanoat, 1,1,3,3-Tetramethylbutylperoxyneodecanoat, tert-Butylperoxy-2-ethylhexanoat, 1,1,3,3-Tetramethylbutylperoxy-2-ethylhexanoat, tert-Amylperoxyneodecanoat, Cumylperoxyneodecanoat, Cumylperoxyneoheptanoat, Cumylperoxypivalat, und deren Mischungen,
und
die Umsetzung bei einer Temperatur im Bereich von 50 bis 110 °C erfolgt, bevorzugt bei einer Temperatur im Bereich von 55 bis 100 °C, und besonders bevorzugt bei einer Temperatur im Bereich von 60 bis 95 °C.

Besonders bevorzugte Ausgestaltungen des erfindungsgemäßen Verfahrens zur Herstellung der Verbindungen der Formel (I) durch Umsetzung einer Verbindung der Formel (II) mit dem Acroleincyanhydrinester der Formel (III), insbesondere zur Herstellung der Verbindungen der Formel (Ia) durch Umsetzung einer Verbindung der Formel (IIa) mit dem Acroleincyanhydrinester der Formel (IIIa), sind dadurch gekennzeichnet, dass
Dosierstrom (D1) 70 - 100 mol.-%, insbesondere bevorzugt 80 - 100 mol.-%, und besonders bevorzugt 90 - 100 mol.-% der Gesamtmenge der in den Dosierströmen (D1) und (D2) insgesamt eingesetzten Menge der radikalbildenden Substanzen (IV) enthält,
Dosierstrom (D2) 0 - 30 mol.-%, insbesondere bevorzugt 0 - 20 mol.-%, und besonders bevorzugt 0 - 10 mol.-% der Gesamtmenge der in den Dosierströmen (D1) und (D2) insgesamt eingesetzten Menge der radikalbildenden Substanzen (IV) enthält,
die Gesamtmenge der Verbindungen (II) bzw. (IIa) und (IV) im Dosierstrom (D1) 80 bis 100 Gew.-% beträgt, bevorzugt 85 bis 100 Gew.-%, besonders bevorzugt 90 bis 100 Gew.-%, jeweils bezogen auf das Gesamtgewicht des Dosierstroms (D1),
das molare Verhältnis der Gesamtmenge der eingesetzten Verbindung der Formel (II) bzw. (IIa) zu der Gesamtmenge der eingesetzten Verbindung der Formel (III) bzw. (IIIa) im Bereich von 5 : 1 bis 2 : 1 liegt,
wobei eine, mehrere oder sämtliche radikalbildenden Substanzen (IV) der Formel (V) entsprechen und ausgewählt sind aus der Gruppe bestehend aus tert-Butylperoxypivalat, tert-Amylperoxypivalat, tert-Butylperoxyneodecanoat, 1,1,3,3-Tetramethylbutylperoxyneodecanoat, tert-Butylperoxy-2-ethylhexanoat, 1,1,3,3-Tetramethylbutylperoxy-2-ethylhexanoat, tert-Amylperoxyneodecanoat, Cumylperoxyneodecanoat, Cumylperoxyneoheptanoat, Cumylperoxypivalat, und deren Mischungen,
und
die Umsetzung bei einer Temperatur im Bereich von 50 bis 110 °C erfolgt, bevorzugt bei einer Temperatur im Bereich von 55 bis 100 °C, und besonders bevorzugt bei einer Temperatur im Bereich von 60 bis 95 °C.

Insbesondere bevorzugte Ausgestaltungen des erfindungsgemäßen Verfahrens zur Herstellung der Verbindungen der Formel (Ia) durch Umsetzung einer Verbindung der Formel (IIa) mit dem Acroleincyanhydrinester der Formel (IIIa), sind dadurch gekennzeichnet, dass
Dosierstrom (D1) 70 - 100 mol.-%, insbesondere bevorzugt 80 - 100 mol.-%, und besonders bevorzugt 90 - 100 mol.-% der Gesamtmenge der in den Dosierströmen (D1) und (D2) insgesamt eingesetzten Menge der radikalbildenden Substanzen (IV) enthält,
Dosierstrom (D2) 0 - 30 mol.-%, insbesondere bevorzugt 0 - 20 mol.-%, und besonders bevorzugt 0 - 10 mol.-% der Gesamtmenge der in den Dosierströmen (D1) und (D2) insgesamt eingesetzten Menge der radikalbildenden Substanzen (IV) enthält,
die Gesamtmenge der Verbindungen (IIa) und (IV) im Dosierstrom (D1) 80 bis 100 Gew.-% beträgt, bevorzugt 85 bis 100 Gew.-%, besonders bevorzugt 90 bis 100 Gew.-%, jeweils bezogen auf das Gesamtgewicht des Dosierstroms (D1),
das molare Verhältnis der Gesamtmenge der eingesetzten Verbindung der Formel (IIa) zu der Gesamtmenge der eingesetzten Verbindung der Formel (IIIa) im Bereich von 5 : 1 bis 2 : 1 liegt,
wobei eine, mehrere oder sämtliche radikalbildenden Substanzen (IV) der Formel (V) entsprechen und ausgewählt sind aus der Gruppe bestehend aus tert-Butylperoxyneodecanoat, 1,1,3,3-Tetramethylbutylperoxyneodecanoat, tert-Butylperoxy-2-ethylhexanoat, 1,1,3,3-Tetramethylbutylperoxy-2-ethylhexanoat, Cumylperoxyneodecanoat, und deren Mischungen, dabei wiederum besonders bevorzugt sind 1,1,3,3-Tetramethylbutylperoxyneodecanoat, tert-Butylperoxyneodecanoat und/oder tert-Butylperoxy-2-ethylhexanoat,
und
die Umsetzung bei einer Temperatur im Bereich von 50 bis 110 °C erfolgt, bevorzugt bei einer Temperatur im Bereich von 55 bis 100 °C, und besonders bevorzugt bei einer Temperatur im Bereich von 60 bis 95 °C.

Insbesondere bevorzugte Ausgestaltungen des erfindungsgemäßen Verfahrens zur Herstellung der Verbindungen der Formel (Ia) durch Umsetzung einer Verbindung der Formel (IIa) mit dem Acroleincyanhydrinester der Formel (IIIa), sind dadurch gekennzeichnet, dass
Dosierstrom (D1) 80 - 100 mol.-%, bevorzugt 90 - 100 mol.-% der Gesamtmenge der in den Dosierströmen (D1) und (D2) insgesamt eingesetzten Menge der radikalbildenden Substanzen (IV) enthält,
Dosierstrom (D2) 0 - 20 mol.-%, bevorzugt 0 - 10 mol.-% der Gesamtmenge der in den Dosierströmen (D1) und (D2) insgesamt eingesetzten Menge der radikalbildenden Substanzen (IV) enthält,
die Gesamtmenge der Verbindungen (IIa) und (IV) im Dosierstrom (D1) 85 bis 100 Gew.-% beträgt, bevorzugt 90 bis 100 Gew.-%, jeweils bezogen auf das Gesamtgewicht des Dosierstroms (D1),
das molare Verhältnis der Gesamtmenge der eingesetzten Verbindung der Formel (IIa) zu der Gesamtmenge der eingesetzten Verbindung der Formel (IIIa) im Bereich von 5 : 1 bis 5 : 2 liegt,
wobei eine, mehrere oder sämtliche radikalbildenden Substanzen (IV) der Formel (V) entsprechen und ausgewählt sind aus der Gruppe bestehend aus tert-Butylperoxyneodecanoat, 1,1,3,3-Tetramethylbutylperoxyneodecanoat, tert-Butylperoxy-2-ethylhexanoat, 1,1,3,3-Tetramethylbutylperoxy-2-ethylhexanoat, Cumylperoxyneodecanoat, und deren Mischungen, dabei wiederum besonders bevorzugt sind 1,1,3,3-Tetramethylbutylperoxyneodecanoat, tert-Butylperoxyneodecanoat und/oder tert-Butylperoxy-2-ethylhexanoat,
und
die Umsetzung bei einer Temperatur im Bereich von 50 bis 110 °C erfolgt, bevorzugt bei einer Temperatur im Bereich von 55 bis 100 °C, und besonders bevorzugt bei einer Temperatur im Bereich von 60 bis 95 °C.

Insbesondere bevorzugte Ausgestaltungen des erfindungsgemäßen Verfahrens zur Herstellung der Verbindungen der Formel (Ia) durch Umsetzung einer Verbindung der Formel (IIa), in der R¹ Methyl bedeutet (und damit der nachfolgend definierten Verbindung der Formel (IIb) entspricht) und R² (C₁-C₆)-Alkyl bedeutet, mit dem Acroleincyanhydrinester der Formel (IIIa), sind dadurch gekennzeichnet, dass
Dosierstrom (D1) 90 - 100 mol.-%, vorzugsweise 95 - 100 mol.-%, bevorzugt 97 - 100 mol.-%, weiter bevorzugt 98 - 100 mol.-% der Gesamtmenge der in den Dosierströmen (D1) und (D2) insgesamt eingesetzten Menge der radikalbildenden Substanzen (IV) enthält,
Dosierstrom (D2) 0 - 10 mol.-%, vorzugsweise 0 - 5 mol.-%, bevorzugt 0 - 3 mol.-%, weiter bevorzugt 0 - 2 mol.-% der Gesamtmenge der in den Dosierströmen (D1) und (D2) insgesamt eingesetzten Menge der radikalbildenden Substanzen (IV) enthält,
die Gesamtmenge der Verbindungen (IIa) und (IV) im Dosierstrom (D1) 85 bis 100 Gew.-% beträgt, bevorzugt 90 bis 100 Gew.-%, jeweils bezogen auf das Gesamtgewicht des Dosierstroms (D1),
das molare Verhältnis der Gesamtmenge der eingesetzten Verbindung der Formel (IIa) zu der Gesamtmenge der eingesetzten Verbindung der Formel (IIIa) im Bereich von 5 : 1 bis 5 : 2 liegt,
wobei eine, mehrere oder sämtliche radikalbildenden Substanzen (IV) der Formel (V) entsprechen und ausgewählt sind aus der Gruppe bestehend aus tert-Butylperoxyneodecanoat, 1,1,3,3-Tetramethylbutylperoxyneodecanoat, tert-Butylperoxy-2-ethylhexanoat, 1,1,3,3-Tetramethylbutylperoxy-2-ethylhexanoat, Cumylperoxyneodecanoat, und deren Mischungen, dabei wiederum besonders bevorzugt sind 1,1,3,3-Tetramethylbutylperoxyneodecanoat, tert-Butylperoxyneodecanoat und/oder tert-Butylperoxy-2-ethylhexanoat,
und
die Umsetzung bei einer Temperatur im Bereich von 55 bis 100 °C erfolgt, besonders bevorzugt bei einer Temperatur im Bereich von 60 bis 95 °C.

Insbesondere bevorzugte Ausgestaltungen des erfindungsgemäßen Verfahrens zur Herstellung der Verbindungen der Formel (Ia) durch Umsetzung einer Verbindung der Formel (IIa), in der R¹ Methyl bedeutet (und damit der nachfolgend definierten Verbindung der Formel (IIb) entspricht) und R² (C₁-C₆)-Alkyl bedeutet, mit dem Acroleincyanhydrinester der Formel (IIIa), sind dadurch gekennzeichnet, dass
Dosierstrom (D1) 90 - 100 mol.-%, vorzugsweise 95 - 100 mol.-%, bevorzugt 97 - 100 mol.-%, weiter bevorzugt 98 - 100 mol.-% der Gesamtmenge der in den Dosierströmen (D1) und (D2) insgesamt eingesetzten Menge der radikalbildenden Substanzen (IV) enthält,
Dosierstrom (D2) 0 - 10 mol.-%, vorzugsweise 0 - 5 mol.-%, bevorzugt 0 - 3 mol.-%, weiter bevorzugt 0 - 2 mol.-% der Gesamtmenge der in den Dosierströmen (D1) und (D2) insgesamt eingesetzten Menge der radikalbildenden Substanzen (IV) enthält,
die Gesamtmenge der Verbindungen (IIa) und (IV) im Dosierstrom (D1) 85 bis 100 Gew.-% beträgt, bevorzugt 90 bis 100 Gew.-%, jeweils bezogen auf das Gesamtgewicht des Dosierstroms (D1),
das molare Verhältnis der Gesamtmenge der eingesetzten Verbindung der Formel (IIa) zu der Gesamtmenge der eingesetzten Verbindung der Formel (IIIa) im Bereich von 9 : 2 bis 5 : 2 liegt,
wobei eine, mehrere oder sämtliche radikalbildenden Substanzen (IV) der Formel (V) entsprechen und ausgewählt sind aus der Gruppe bestehend aus tert-Butylperoxyneodecanoat, 1,1,3,3-Tetramethylbutylperoxyneodecanoat, tert-Butylperoxy-2-ethylhexanoat, 1,1,3,3-Tetramethylbutylperoxy-2-ethylhexanoat, Cumylperoxyneodecanoat, und deren Mischungen, dabei wiederum besonders bevorzugt sind 1,1,3,3-Tetramethylbutylperoxyneodecanoat, tert-Butylperoxyneodecanoat und/oder tert-Butylperoxy-2-ethylhexanoat,
und
die Umsetzung bei einer Temperatur im Bereich von 55 bis 100 °C erfolgt, besonders bevorzugt bei einer Temperatur im Bereich von 60 bis 95 °C.

Insbesondere bevorzugte Ausgestaltungen des erfindungsgemäßen Verfahrens zur Herstellung der Verbindungen der Formel (Ia) durch Umsetzung einer Verbindung der Formel (IIa), in der R¹ Methyl bedeutet (und damit der nachfolgend definierten Verbindung der Formel (IIb) entspricht) und R² (C₁-C₆)-Alkyl bedeutet, mit dem Acroleincyanhydrinester der Formel (IIIa), sind dadurch gekennzeichnet, dass
Dosierstrom (D1) 95 - 100 mol.-%, bevorzugt 97 - 100 mol.-%, weiter bevorzugt 98 - 100 mol.-% der Gesamtmenge der in den Dosierströmen (D1) und (D2) insgesamt eingesetzten Menge der radikalbildenden Substanzen (IV) enthält,
Dosierstrom (D2) 0 - 5 mol.-%, bevorzugt 0 - 3 mol.-%, weiter bevorzugt 0 - 2 mol.-% der Gesamtmenge der in den Dosierströmen (D1) und (D2) insgesamt eingesetzten Menge der radikalbildenden Substanzen (IV) enthält,
die Gesamtmenge der Verbindungen (IIa) und (IV) im Dosierstrom (D1) 90 bis 100 Gew.-% beträgt, bezogen auf das Gesamtgewicht des Dosierstroms (D1),
das molare Verhältnis der Gesamtmenge der eingesetzten Verbindung der Formel (IIa) zu der Gesamtmenge der eingesetzten Verbindung der Formel (IIIa) im Bereich von 9 : 2 bis 5 : 2 liegt,
wobei sämtliche radikalbildenden Substanzen (IV) der Formel (V) entsprechen und ausgewählt sind aus der Gruppe bestehend aus 1,1,3,3-Tetramethylbutylperoxyneodecanoat, tert-Butylperoxyneodecanoat, tert-Butylperoxy-2-ethylhexanoat und deren Mischungen,
und
die Umsetzung bei einer Temperatur im Bereich von 55 bis 100 °C erfolgt, besonders bevorzugt bei einer Temperatur im Bereich von 60 bis 95 °C.

Insbesondere bevorzugte Ausgestaltungen des erfindungsgemäßen Verfahrens zur Herstellung der Verbindungen der Formel (Ia) durch Umsetzung einer Verbindung der Formel (IIa), in der R¹ Methyl bedeutet (und damit der nachfolgend definierten Verbindung der Formel (IIb) entspricht) und R² (C₁-C₆)-Alkyl bedeutet, mit dem Acroleincyanhydrinester der Formel (IIIa), sind dadurch gekennzeichnet, dass
Dosierstrom (D1) 97 - 100 mol.-%, bevorzugt 98 - 100 mol.-% der Gesamtmenge der in den Dosierströmen (D1) und (D2) insgesamt eingesetzten Menge der radikalbildenden Substanzen (IV) enthält,
Dosierstrom (D2) 0 - 3 mol.-%, bevorzugt 0 - 2 mol.-% der Gesamtmenge der in den Dosierströmen (D1) und (D2) insgesamt eingesetzten Menge der radikalbildenden Substanzen (IV) enthält,
die Gesamtmenge der Verbindungen (IIa) und (IV) im Dosierstrom (D1) 90 bis 100 Gew.-% beträgt, bezogen auf das Gesamtgewicht des Dosierstroms (D1),
das molare Verhältnis der Gesamtmenge der eingesetzten Verbindung der Formel (IIa) zu der Gesamtmenge der eingesetzten Verbindung der Formel (IIIa) im Bereich von 9 : 2 bis 5 : 2 liegt,
wobei sämtliche radikalbildenden Substanzen (IV) der Formel (V) entsprechen und ausgewählt sind aus der Gruppe bestehend aus 1,1,3,3-Tetramethylbutylperoxyneodecanoat, tert-Butylperoxyneodecanoat, tert-Butylperoxy-2-ethylhexanoat und deren Mischungen,
und
die Umsetzung bei einer Temperatur im Bereich von 60 bis 95 °C erfolgt.

Das erfindungsgemäße Verfahren kann sowohl in diskontinuierlicher Prozessführung (z.B. in semi-Batch Fahrweise) als auch in kontinuierlicher Prozessführung (z.B. im kontinuierlich betriebenen Rührkessel) durchgeführt werden.

Unter einer kontinuierlichen Prozessführung im Sinne der vorliegenden Erfindung ist zu verstehen, dass Verbindungen (d.h. Edukten wie Verbindungen der Formel (II) und (III)) in den Reaktor eingebracht werden (Eintrag / Zulauf), und gleichzeitig, aber davon räumlich getrennt, Verbindungen (d.h. Produkte wie Verbindungen der Formel (I)) aus dem Reaktor ausgebracht werden (Austrag / Ablauf).

Bei einer diskontinuierlichen Prozessführung hingegen erfolgen die Schritte Eintrag von Edukten (d.h. von Edukten wie Verbindungen der Formel (II) und (III)), Umsetzung (d.h. Reaktion der Edukte), und Austrag der Produkte (d.h. Produkte wie Verbindungen der Formel (I)) aus dem Reaktor zeitlich nacheinander oder nur in einzelnen Abschnitten überlappend.

Das erfindungsgemäße Verfahren wird bevorzugt in der Weise durchgeführt, dass die Dosierung der Dosierströme (D1) und (D2) in den Reaktor im Wesentlichen kontinuierlich, vorzugsweise kontinuierlich erfolgt.

Eine solche im Wesentlichen kontinuierliche, oder vorzugsweise kontinuierliche, Prozessführung ist von wirtschaftlichem Vorteil, da beispielsweise unproduktive Reaktorzeiten infolge von Befüllungs- und Entleerungsprozessen und verlängerte Reaktionszeiten infolge sicherheitstechnischer Gründe, reaktorspezifischer Wärmetauschleistungen sowie Aufheiz- und Abkühlungsprozesse, wie sie bei semi-Batch-Verfahren und Batch-Verfahren auftreten, vermieden bzw. minimiert werden können.

Das erfindungsgemäße Verfahren wird vorzugsweise unter Inertisierung, bevorzugt in einer Schutzgasatmosphäre durchgeführt. Bevorzugte Schutzgase sind dabei Stickstoff bzw. Argon.

Es ist ferner möglich, das erfindungsgemäße Verfahren unter Überdruck oder unter vermindertem Druck durchzuführen.

Das erfindungsgemäße Verfahren kann in einem optionalen Verdünnungsmittel durchgeführt werden.

Als optionale Verdünnungsmittel sind grundsätzlich verschiedene organische Lösungsmittel einsetzbar, vorzugsweise Toluol, Xylol, Chlorbenzol, Dichlorbenzol, Dimethylformamid (DMF), Dimethylacetamid, *N*-Methyl-2-pyrrolidon (NMP), oder Mischungen dieser organischen Lösungsmittel. Bevorzugt wird das erfindungsgemäße Verfahren ohne solche weitere optionale Lösungsmittel durchgeführt.

Es kann allerdings vorteilhaft sein, das erfindungsgemäße Verfahren in bereits vorher gebildetem Reaktionsprodukt der Formel (I), (Ia) bzw. (Ib) als Verdünnungsmittel durchzuführen.

Insbesondere bei kontinuierlicher Fahrweise ist es vorteilhaft, das erfindungsgemäße Verfahren in bereits vorher gebildetem Reaktionsprodukt der Formel (I), (Ia) bzw. (Ib) oder in einem Gemisch von Reaktionsprodukt der Formel (I), (Ia) bzw. (Ib) und Edukt der Formel (II) bzw. (IIa) als Verdünnungsmittel durchzuführen.

Die Reinheit der gewünschten Produkte der Formel (I) nach Reinigung, zum Beispiel nach destillativem Entfernen eines Überschusses der Komponente (II) bzw. (IIa), liegt regelmäßig bei mehr als 95%. Ein vorzugsweise zurückgewonnener Überschuss der Ausgangsverbindung (II) bzw. (IIa) kann anschließend ohne weitere Reinigung wieder in dieselbe Reaktion eingesetzt werden.

Dies gilt insbesondere für die Verbindung der Formel (Ib) mit R² = n-Butyl, die nach dem erfindungsgemäßen Verfahren durch Umsetzung des phosphorhaltigen Edukts (IIa) mit R¹ = Methyl und R² = n-Butyl mit Acroleincyanhydrin-O-acetat der Formel (IIIa) erhalten wird.

Bei Glufosinat-Salzen im Rahmen der vorliegenden Erfindung handelt es sich vorzugsweise um Ammonium-Salze, Phosphonium-Salze, Sulfonium-Salze, Alkali-Salze und Erdalkali-Salze von Glufosinat.

Insbesondere bevorzugt im Rahmen der vorliegenden Erfindung sind Glufosinat, Glufosinat-Natrium bzw. Glufosinat-Ammonium.

In einem weiteren Aspekt betrifft die vorliegende Erfindung die Herstellung von Glufosinat 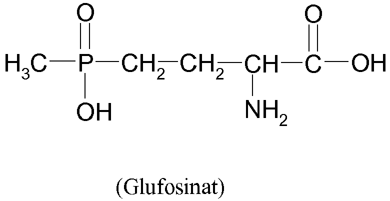 oder Glufosinat-Salzen (dabei vorzugsweise Glufosinat-Ammonium), dadurch gekennzeichnet, dass in diesem Verfahren eine Verbindung der Formel (Ib) eingesetzt wird 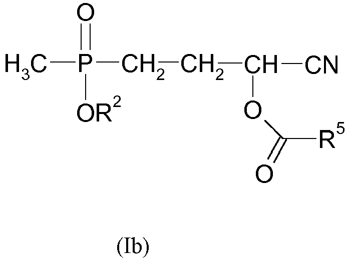
wobei R² die vorstehend erfindungsgemäß definierte Bedeutung hat, bevorzugt die vorstehend als bevorzugt definierte Bedeutung, und besonders bevorzugt die vorstehend als besonders bevorzugt definierte Bedeutung, und
R⁵ die vorstehend genannte Bedeutung hat, und vorzugsweise Methyl bedeutet, und
die Herstellung der Verbindung der Formel (Ib) gemäß einem erfindungsgemäß definierten Verfahren erfolgt.

In einem bevorzugten Aspekt betrifft die vorliegende Erfindung die Herstellung von Glufosinat und/oder Glufosinat-Salzen 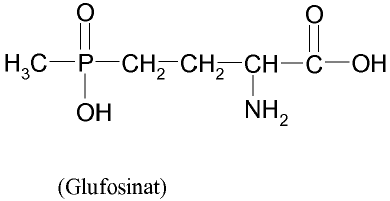 gekennzeichnet durch Umsetzung einer Verbindung der Formel (Ib), durch folgenden Schritt:
Umsetzung einer Verbindung der Formel (IIb) 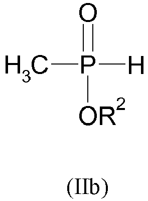 wobei
R² (C₁-C₆)-Alkyl bedeutet, bevorzugt (C₄-C₅)-Alkyl, und besonders bevorzugt n-Butyl oder n-Pentyl bedeutet,
mit Acroleincyanhydrin-O-acetat der Formel (IIIa) 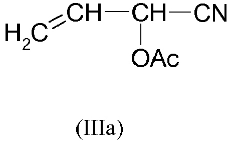 wobei die Umsetzung von (IIb) mit (IIIa) nach dem oben beschriebenen erfindungsgemäßen Verfahren erfolgt, bevorzugt in einer der als bevorzugt beschriebenen Ausgestaltungen, und besonders bevorzugt in einer der als besonders bevorzugt beschriebenen Ausgestaltungen.

Das erfindungsgemäße Verfahren zur Herstellung von Glufosinat und/oder Glufosinat-Salzen erfolgt im Weiteren vorzugsweise durch Umsetzung einer Verbindung der Formel (Ib) mit NH₃ zu Verbindung (VI) 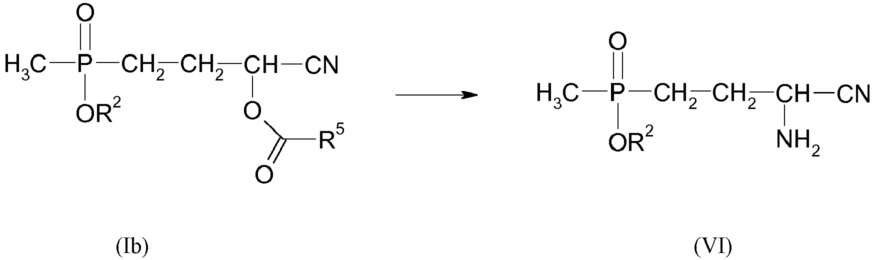 wobei R² und R⁵ jeweils die oben genannte Bedeutung haben,
und anschließender Hydrolyse von Verbindung (VI) zu Glufosinat bzw. dessen Salzen.

Das erfindungsgemäße Verfahren zur Herstellung von Glufosinat und/oder Glufosinat-Salzen kann in ähnlicher Weise erfolgen, wie beispielsweise in US 4,521,348 beschrieben.

Schließlich betrifft die vorliegende Erfindung auch die Verwendung einer gemäß einem erfindungsgemäßen Verfahren hergestellten Verbindung der Formel (I) bzw. (Ib), wie vorstehend definiert, zur Herstellung von Glufosinat bzw. von Glufosinat-Salzen, insbesondere von Glufosinat, Glufosinat-Natrium oder Glufosinat-Ammonium.

Ferner betrifft die vorliegende Erfindung ein Verfahren zur Herstellung von Glufosinat bzw. von Glufosinat-Salzen, insbesondere von Glufosinat, Glufosinat-Natrium oder Glufosinat-Ammonium, umfassend die folgenden Schritte (a) und (b):
(a) Herstellung einer gemäß einem erfindungsgemäßen Verfahren hergestellten Verbindung der Formel (I) bzw. (Ib), wie vorstehend definiert,
(b) Verwendung der in Schritt (a) hergestellten Verbindung der Formel (I) bzw. (Ib) zur Herstellung von Glufosinat bzw. von Glufosinat-Salzen, insbesondere von Glufosinat, Glufosinat-Natrium oder Glufosinat-Ammonium.

Die nachfolgenden Beispiele veranschaulichen die vorliegende Erfindung.

### Beispiele:

Sofern nicht anders angegeben beziehen sich alle Angaben auf das Gewicht.

Verwendete Abkürzungen:
- MPE:: Methanphosphonigsäuremono-n-butylester
- ACA:: Acroleincyanhydrinacetat
- ACM:: n-Butyl (3-cyano-3-acetoxypropyl)methylphosphinat

### Beispiel 1: n-Butyl (3-cyano-3-acetoxypropyl)methylphosphinat (ACM)

### Diskontinuierliche Fahrweise

Ein temperierbarer, zylindrischer Glasreaktor wurde mit einer Teilmenge des erforderlichen MPEs befüllt, um das Rührwerk ausreichend zu bedecken, und der Reaktorinhalt auf Reaktionstemperatur (typischerweise 85°C) gebracht. Bei den Versuchen mit Umpump/Umlaufkreislauf wurde außerdem noch der Umlaufkreislauf inkl. der dazugehörigen Pumpe mit MPE befüllt. Die Durchmischung des Reaktorinhalts wurde durch einen 6-Blatt-Scheibenrührer in Kombination mit vier Strömungsbrechern bewerkstelligt. Der Reaktorinhalt wurde stets mit Stickstoff überlagert und ohne Überdruck betrieben.

5 Minuten vor Beginn der Dosierung der Edukte in den Reaktor wurde eine kleine Menge an Initiator (0,9-1,0 mL, entsprechend ca. 0,8-0,9 g) in das vorgelegte, auf Reaktionstemperatur erhitzte (und ggf. auch durch den Umlaufkreislauf zirkulierende) MPE injiziert, um einen zuverlässigen Start der Umsetzung (d.h. ein zuverlässiges Anspringen) zu gewährleisten. Die Zeitspanne von 5 Minuten entspricht bei 85°C etwa der Halbwertszeit des Radikalstarters tert.-Butylperneodecanoat. Zum Ende der Dosierzeit von 4 Stunden (d.h. mehr als 40 Halbwertszeiten des eingesetzten Radikalstarters), war die vorab injizierte Menge an tert.-Butylperneodecanoat auf <10⁻¹² Teile der Ausgangsmenge abgefallen und damit ohne nennenswerte weitere Relevanz für eventuelle nachfolgende Versuche (wie z.B. der ACM-Herstellung in kontinuierlicher Fahrweise).

Nachfolgend wurden die Dosierströme (D1) und (D2) separat in den Reaktor dosiert, bis der gewünschte Füllstand erreicht war, unter Berücksichtigung der jeweiligen Stöchiometrien.

142,0 g MPE (Reinheit: 98%ig) wurden vorgelegt und auf 85°C erhitzt. 5 min vor Beginn der Dosierströme (D1) und (D2) wurde 1,0 mL (ca. 0,9 g) des Radikalstarters tert.-Butylperneodecanoat (Reinheit: 98%ig) zugegeben. Anschließend wurden folgende Dosierströme (D1) und (D2) gleichzeitig über einen Zeitraum von 4,0 h in den Reaktor eindosiert:
Dosierstrom (D1) war eine Mischung aus MPE (102,1 g, Reinheit: 98%ig) und tert.-Butylperneodecanoat (3,0 g, Reinheit: 98%ig), Dosierstrom (D2) bestand aus 57,0 g ACA (Reinheit: 99%ig).

Die Konzentration des Radikalstarters betrug damit 1,0 Gew.-%, bezogen auf die Gesamtmischung.

Das eingesetzte ACA war vollständig umgesetzt worden; die Ausbeute für die Umsetzung von ACA zu ACM betrug 98-99%. Nach Ablauf der Dosierzeit hatte der diskontinuierliche Ansatz seinen Endpunkt erreicht, und es konnte beispielsweise auf kontinuierliche Fahrweise umgestellt werden (siehe nachfolgendes Beispiel 2).

### Beispiel 2: n-Butyl (3-cyano-3-acetoxypropyl)methylphosphinat (ACM)

### Kontinuierliche Fahrweise

In den Reaktor vorgelegt wurde eine Mischung, die nach der diskontinuierlichen Fahrweise gemäß vorstehendem Beispiel 1 hergestellt wurde. Die Reaktionsbedingungen und die apparativen Parameter entsprachen denen aus Beispiel 1.Bei einer Reaktionstemperatur von 85°C wurden dann simultan die Dosierströme (D1) und (D2) separat in den Reaktor eindosiert.

Bei 85°C wurde dem Reaktor mit Dosierstrom (D1) eine Mischung aus MPE und Radikalstarter tert.-Butylperneodecanoat mit 63 mL/h und mit Dosierstrom (D2) ACA mit 14 mL/h zugegeben; dabei wurde der Gehalt des Radikalstarters im MPE so gewählt (1,2 Gew.-%), dass in der Gesamtmischung im Reaktor ein Gehalt von 1,0 Gew.-% an Radikalstarter erreicht wurde. Die Durchmischung des Reaktorinhalts wurde durch einen 6-Blatt-Scheibenrührer in Kombination mit vier Strömungsbrechern bewerkstelligt.

Entsprechend der zugeführten Volumenströme, wurde aus dem Reaktor ein ausreichend dimensionierter Volumenstrom der Reaktormischung entnommen, um das Füllvolumen des Reaktors konstant zu halten. Aus dem Füllvolumen des Reaktors und den zugeführten bzw. abgeführten Volumenströmen ergab sich eine mittlere hydrodynamische Verweilzeit von 4,0 h im Reaktor.

Nach Erreichen des stationären Zustands wurden Proben vom Reaktorinhalt entnommen und eine Ausbeute von 95-96% für die Umsetzung von ACA zu ACM bestimmt.

### Beispiel 3: n-Butyl (3-cyano-3-acetoxypropyl)methylphosphinat (ACM)

Geräte: 500 ml Mantelrührgefäß mit Thermometer, Impeller-Rührer und Boden-Auslassventil; zwei Pumpen.

In einem 500 ml Mantelrührgefäß mit Thermometer, Impeller-Rührer (400 UPM) und geschlossenem Boden-Auslassventil wurden unter Inertisierung mit Stickstoff wurden 100,0 g eines Reaktionsgemischs aus einem früheren Reaktionsansatz (Zusammensetzung laut GC: 36% MPE und 56,7% ACM) vorgelegt und auf 85°C erwärmt. Dazu wurde 1,0 g (0,00405 mol) des Radikalstarters t-Butylperoxyneodecanoat zugegeben.

Anschließend wurden simultan folgende Dosierströme (D1) und (D2) von zwei Waagen gleichmäßig mittels zweier Pumpen über einen Zeitraum von 135 Minuten in das Mantelrührgefäß eindosiert, wobei die Innentemperatur bei 85°C gehalten wurde:
Dosierstrom (D1) war ein Gemisch aus 25,0 g (0,18 mol) MPE ((Reinheit 99%ig) und 0,9 g des Radikalstarters t-Butylperoxyneodecanoat (Reinheit: 98%ig), und
Dosierstrom (D2) bestand aus 10,0 g (0,079 mol) ACA (Reinheit 99%ig).

Dieser Dosiervorgang wurde anschließend noch einmal unter den gleichen Bedingungen mit denselben Mengen an den identisch zusammengesetzten Dosierströmen (D1) und (D2) wiederholt.

Das nach beendeter Reaktion erhaltene Reaktionsgemisch war klar und blassgelb, und enthielt gemäß Analyse kein ACA mehr. Nach Abkühlen wurde das Reaktionsgemisch (171,5 g) analysiert (GC und NMR), es enthielt 39% MPE und 54,7% ACM. Die Ausbeute entsprach 96% der Theorie, bezogen auf die eingesetzte Menge an ACA. Die Trennung von MPE und ACM erfolgte anschließend über eine Kurzweg-Dünnschichtdestillation im Vakuum.

### Vergleichsbeispiel 1: n-Butyl (3-cyano-3-acetoxypropyl)methylphosphinat (ACM)

Sofern nachfolgend nicht anders angegeben, wurden die in Beispiel 1 beschriebenen Materialien, Bedingungen und Geräte verwendet.

Unter Stickstoff wurden 97,7 g MPE (Reinheit: 98%ig; 0,703 mol) vorgelegt und auf 85°C erhitzt. Nach Zugabe eines Tropfens des Radikalstarters t-Butylperoxyneodecanoat wurde dann bei gleicher Temperatur über eine Spritzenpumpe unter starkem Rühren eine Mischung von 23,6 g ACA (Reinheit 98%ig; 0,1848 mol) und 1,2 g t-Butylperoxyneodecanoat (0,005 mol) innerhalb von 4 Stunden mit konstanter Rate in den Reaktor dosiert. Nach einer Nachreaktionszeit von 15 min wurde auf 20°C abgekühlt.

Gemäß GC-Analyse waren in dem erhaltenen Reaktionsgemisch 36,1% an gewünschtem Produkt ACM enthalten, entsprechend 91,6% der Theorie

## Patentansprüche

1. Verfahren zur Herstellung einer Verbindung der Formel (I) 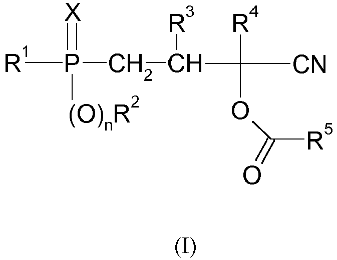 **dadurch gekennzeichnet, dass** eine Verbindung der Formel (II) 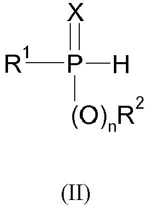 mit einer Verbindung der Formel (III) 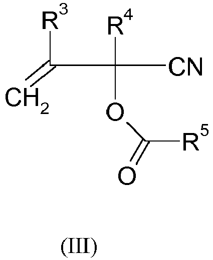 wobei jeweils gilt:
R¹ bedeutet (C₁-C₁₂)-Alkyl, (C₁-C₁₂)-Haloalkyl, (C₆-C₁₀)-Aryl, (C₆-C₁₀)-Haloaryl, (C₇-C₁₀)-Aralkyl, (C₇-C₁₀)-Haloaralkyl, (C₄-C₁₀)-Cycloalkyl oder (C₄-C₁₀)-Halocycloalkyl,
R² bedeutet (C₁-C₁₂)-Alkyl, (C₁-C₁₂)-Haloalkyl, (C₆-C₁₀)-Aryl, (C₆-C₁₀)-Haloaryl, (C₇-C₁₀)-Aralkyl, (C₇-C₁₀)-Haloaralkyl, (C₄-C₁₀)-Cycloalkyl oder (C₄-C₁₀)-Halocycloalkyl,
R³ und R⁴ bedeuten jeweils unabhängig voneinander Wasserstoff, (C₁-C₄)-Alkyl, Phenyl oder Benzyl,
R⁵ bedeutet (C₁-C₁₂)-Alkyl, (C₁-C₁₂)-Haloalkyl, (C₆-C₁₀)-Aryl, (C₆-C₁₀)-Haloaryl, (C₇-C₁₀)-Aralkyl, (C₇-C₁₀)-Haloaralkyl, (C₄-C₁₀)-Cycloalkyl oder (C₄-C₁₀)-Halocycloalkyl,
X bedeutet Sauerstoff oder Schwefel, und
n ist 0 oder 1,
in Gegenwart einer oder mehrerer radikalbildender Substanzen (IV) umgesetzt wird, wobei
ein Dosierstrom (D1) in den Reaktor dosiert wird, welcher eine oder mehrere Verbindungen der Formel (II) und eine oder mehrere radikalbildende Substanzen (IV) enthält, und wobei Dosierstrom (D1) 25 - 100 mol.-% der Gesamtmenge der in die Umsetzung insgesamt eingesetzten Menge der radikalbildenden Substanzen (IV) enthält.

2. Verfahren nach Anspruch 1, **dadurch gekennzeichnet, dass** zwei separate Dosierströme (D1) und (D2) in den Reaktor dosiert werden, und diese Dosierströme (D1) und (D2) folgende Zusammensetzung aufweisen:
Dosierstrom (D1) enthält eine oder mehrere Verbindungen der Formel (II) und eine oder mehrere radikalbildende Substanzen (IV), wobei Dosierstrom (D1) 25 - 100 mol.-% der Gesamtmenge der in die Umsetzung insgesamt eingesetzten Menge der radikalbildenden Substanzen (IV) enthält,
und
Dosierstrom (D2) enthält eine oder mehrere Verbindungen der Formel (III), sowie gegebenenfalls eine oder mehrere Verbindungen der Formel (II), und gegebenenfalls eine oder mehrere radikalbildende Substanzen (IV).

3. Verfahren nach Anspruch 1 oder 2, **dadurch gekennzeichnet, dass** Dosierstrom (D1) 30 - 100 mol.-% der Gesamtmenge der in die Umsetzung insgesamt eingesetzten Menge der radikalbildenden Substanzen (IV) enthält, vorzugsweise 40 - 100 mol.-%,bevorzugt 50 - 100 mol.-%, weiter bevorzugt 60 - 100 mol.-%, noch weiter bevorzugt 70 - 100 mol.-%, besonders bevorzugt 80 - 100 mol.-%, insbesondere bevorzugt 90 - 100 mol.-% und am meisten bevorzugt 95 - 100 mol.-%.

4. Verfahren nach einem der Ansprüche 2 oder 3, **dadurch gekennzeichnet, dass** Dosierstrom (D1) 90 - 100 Gew.-%, bevorzugt (D1) 95 - 100 Gew.-%, weiter bevorzugt 100 Gew.-% der Gesamtmenge der in den Dosierströmen (D1) und (D2) insgesamt eingesetzten Menge an Verbindungen der Formel (II) enthält.

5. Verfahren nach einem der Ansprüche 2 bis 4, **dadurch gekennzeichnet, dass**
Dosierstrom (D2) 80 - 100 Gew.-%, bevorzugt 90 - 100 Gew.-%, bevorzugt 95 - 100 Gew.-%, insbesondere bevorzugt 100 Gew.-% der Gesamtmenge der in den Dosierströmen (D1) und (D2) insgesamt eingesetzten Menge Verbindungen der Formel (III) enthält.

6. Verfahren nach einem der Ansprüche 2 bis 5, **dadurch gekennzeichnet, dass**
Dosierstrom (D1) 40 - 100 mol.-%, vorzugsweise 50 - 100 mol.-%, bevorzugt 60 - 100 mol.-%, weiter bevorzugt 70 - 100 mol.-%, insbesondere bevorzugt 80 - 100 mol.-%, und besonders bevorzugt 90 - 100 mol.-% der Gesamtmenge der in den Dosierströmen (D1) und (D2) insgesamt eingesetzten Menge der radikalbildenden Substanzen (IV) enthält,
und/oder
Dosierstrom (D2) 0 - 60 mol.-%, vorzugsweise 0 - 50 mol.-%, bevorzugt 0 - 40 mol.-%, weiter bevorzugt 0 - 30 mol.-%, insbesondere bevorzugt 0 - 20 mol.-%, und besonders bevorzugt 0 - 10 mol.-% der Gesamtmenge der in den Dosierströmen (D1) und (D2) insgesamt eingesetzten Menge der radikalbildenden Substanzen (IV) enthält.

7. Verfahren nach einem der Ansprüche 1 bis 6, **dadurch gekennzeichnet, dass** die Gesamtmenge der Verbindungen (II) und (IV) im Dosierstrom (D1) 75 bis 100 Gew.-% beträgt, bevorzugt 80 bis 100 Gew.-%, weiter bevorzugt 85 bis 100 Gew.-%, besonders bevorzugt 90 bis 100 Gew.-%, jeweils bezogen auf das Gesamtgewicht des Dosierstroms (D1).

8. Verfahren nach einem der Ansprüche 2 bis 7, **dadurch gekennzeichnet, dass** die Dosierströme (D1) und (D2) überwiegend simultan, vorzugsweise simultan in den Reaktor dosiert werden.

9. Verfahren nach einem der Ansprüche 2 bis 8, **dadurch gekennzeichnet, dass** die Dosierung der Dosierströme (D1) und (D2) in den Reaktor im Wesentlichen kontinuierlich, vorzugsweise kontinuierlich erfolgt.

10. Verfahren nach einem der Ansprüche 1 bis 9, **dadurch gekennzeichnet, dass** eine, mehrere oder sämtliche radikalbildenden Substanzen (IV) der Formel (V) entsprechen 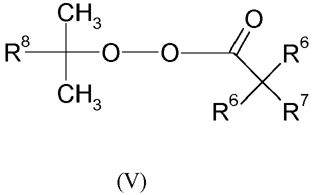 wobei
R⁶ unabhängig voneinander Wasserstoff, (C₁-C₁₀)-Alkyl bedeutet, vorzugsweise (C₁-C₆)-Alkyl, bevorzugt (C₁-C₄)-Alkyl,
R⁷ Wasserstoff oder (C₁-C₁₀)-Alkyl bedeutet, vorzugsweise Wasserstoff oder (C₁-C₆)-Alkyl, bevorzugt Wasserstoff oder (C₁-C₄)-Alkyl,
und
R⁸ Methyl, Ethyl, 2,2-Dimethylpropyl oder Phenyl bedeutet.

11. Verfahren nach einem der Ansprüche 1 bis 10, **dadurch gekennzeichnet, dass** die Umsetzung bei einer Temperatur im Bereich von 40 bis 120 °C erfolgt, bevorzugt bei einer Temperatur im Bereich von 50 bis 110 °C, weiter bevorzugt bei einer Temperatur im Bereich von 55 bis 100 °C, und besonders bevorzugt bei einer Temperatur im Bereich von 60 bis 95 °C.

12. Verfahren nach einem der Ansprüche 1 bis 11, **dadurch gekennzeichnet, dass** das molare Verhältnis der Gesamtmenge der eingesetzten Verbindung der Formel (II) zu der Gesamtmenge der eingesetzten Verbindung der Formel (III) im Bereich von 8 : 1 bis 1 : 1 liegt, vorzugsweise im Bereich von 5 : 1 bis 2 : 1.

13. Verfahren nach einem der Ansprüche 1 bis 12, **dadurch gekennzeichnet, dass**
eine der Verbindungen oder die Verbindung der Formel (II) der Formel (IIa) entspricht 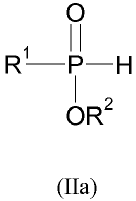 wobei
R¹ (C₁-C₆)-Alkyl, (C₁-C₆)-Haloalkyl, (C₆-C₈)-Aryl, (C₆-C₈)-Haloaryl, (C₇-C₁₀)-Aralkyl, (C₇-C₁₀)-Haloaralkyl, (C₅-C₈)-Cycloalkyl oder (C₅-C₈)-Halocycloalkyl bedeutet, und
R² (C₁-C₆)-Alkyl, (C₁-C₆)-Haloalkyl, (C₆-C₈)-Aryl, (C₆-C₈)-Haloaryl, (C₇-C₁₀)-Aralkyl, (C₇-C₁₀)-Haloaralkyl, (C₅-C₈)-Cycloalkyl oder (C₅-C₈)-Halocycloalkyl bedeutet,
und
eine der Verbindungen oder die Verbindung der Formel (III) der Formel (IIIa) entspricht

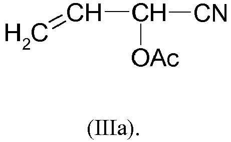

14. Verfahren zur Herstellung von Glufosinat 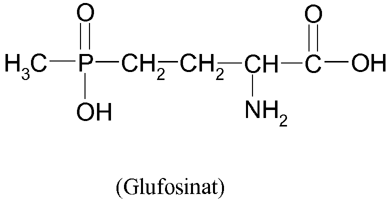 oder Glufosinat-Salzen, **dadurch gekennzeichnet, dass** in diesem Verfahren eine Verbindung der Formel (Ib) eingesetzt wird 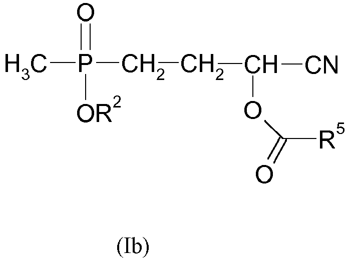
wobei R² die in Anspruch 1 oder Anspruch 13 genannte Bedeutung hat,
R⁵ die in Anspruch 1 genannte Bedeutung hat oder Methyl bedeutet, und
die Herstellung der Verbindung der Formel (Ib) gemäß einem in den Ansprüchen 1 bis 13 definierten Verfahren erfolgt.

15. Verfahren zur Herstellung von Glufosinat bzw. von Glufosinat-Salzen, insbesondere von Glufosinat, Glufosinat-Natrium oder Glufosinat-Ammonium, umfassend die folgenden Schritte (a) und (b):
(a) Herstellung einer gemäß einem in den Ansprüchen 1 bis 13 definierten Verfahren hergestellten Verbindung der Formel (I) bzw. (Ib), wie in Anspruch 1, Anspruch 13 bzw. Anspruch 14 definiert,
(b) Verwendung der in Schritt (a) hergestellten Verbindung der Formel (I) bzw. (Ib) zur Herstellung von Glufosinat bzw. von Glufosinat-Salzen, insbesondere von Glufosinat, Glufosinat-Natrium oder Glufosinat-Ammonium.

## Claims

1. A process for producing a compound of formula (I) 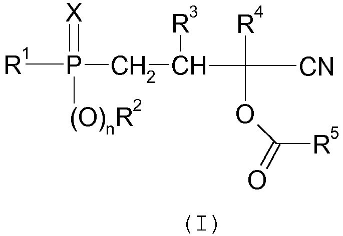 wherein a compound of formula (II) 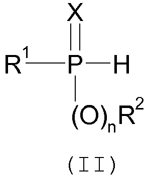 is reacted with a compound of formula (III) 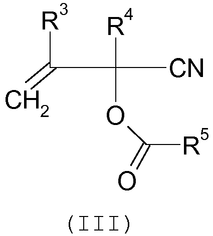 where in each case:
R¹ represents (C₁-C₁₂)-alkyl, (C₁-C₁₂)-haloalkyl, (C₆-C₁₀)-aryl, (C₆-C₁₀)-haloaryl, (C₇-C₁₀)-aralkyl, (C₇-C₁₀)-haloaralkyl, (C₄-C₁₀)-cycloalkyl or (C₄-C₁₀)-halocycloalkyl,
R² represents (C₁-C₁₂)-alkyl, (C₁-C₁₂)-haloalkyl, (C₆-C₁₀)-aryl, (C₆-C₁₀)-haloaryl, (C₇-C₁₀)-aralkyl, (C₇-C₁₀)-haloaralkyl, (C₄-C₁₀)-cycloalkyl or (C₄-C₁₀)-halocycloalkyl,
R³ and R⁴ each independently of one another represent hydrogen, (C₁-C₄)-alkyl, phenyl or benzyl, R⁵ represents (C₁-C₁₂)-alkyl, (C₁-C₁₂)-haloalkyl, (C₆-C₁₀)-aryl, (C₆-C₁₀)-haloaryl, (C₇-C₁₀)-aralkyl, (C₇-C₁₀)-haloaralkyl, (C₄-C₁₀)-cycloalkyl or (C₄-C₁₀)-halocycloalkyl,
X represents oxygen or sulphur and
n is 0 or 1,
in the presence of one or more free-radical-forming substances (IV), where a metered stream (D1) is metered into the reactor, which comprises one or more compounds of formula (II) and one or more free-radical-forming substances (IV), and where metered stream (D1) comprises 25 - 100 mol% of the entirety of the amount of the free-radical-forming substances (IV) altogether employed in the reaction.

2. The process according to claim 1, wherein two separate metered streams (D1) and (D2) are metered into the reactor and these metered streams (D1) and (D2) have the following composition:
metered stream (D1) comprises one or more compounds of formula (II) and one or more free-radical-forming substances (IV), where metered stream (D1) comprises 25-100 ml% of the amount of the free-radical-forming substances (IV) altogether employed in the reaction
and
metered stream (D2) comprises one or more compounds of formula (III) and also optionally one or more compounds of formula (II) and optionally one or more free-radical-forming substances (IV).

3. The process according to claim 1 or 2, wherein metered stream (D1) comprises 30 - 100 mol% of the entirety of the amount of the free-radical-forming substances (IV) altogether employed in the reaction, by preference 40 - 100 mol%, preferably 50 - 100 mol%, more preferably 60 - 100 mol%, yet more preferably 70 - 100 mol%, particularly preferably 80 - 100 mol%, especially preferably 90 - 100 mol% and most preferably 95 - 100 mol%.

4. The process according to either of claims 2 or 3, wherein metered stream (D1) comprises 90 - 100 wt%, preferably (D1) comprises 95 - 100 wt%, more preferably 100 wt%, of the entirety of the amount of compounds of formula (II) altogether employed in the metered streams (D1) and (D2).

5. The process according to any of claims 2 to 4, wherein
metered stream (D2) comprises 80 - 100 wt%, preferably 90 - 100 wt%, preferably 95 - 100 wt%, especially preferably 100 wt%, of the entirety of the amount of compounds of formula (III) altogether employed in the metered streams (D1) and (D2).

6. The process according to any of claims 2 to 5, wherein
metered stream (D1) comprises 40 - 100 mol%, by preference 50 - 100 mol%, preferably 60 - 100 mol%, more preferably 70 - 100 mol%, especially preferably 80 - 100 mol% and particularly preferably 90 - 100 mol% of the entirety of the amount of the free-radical-forming substances (IV) altogether employed in the metered streams (D1) and (D2)
and/or
metered stream (D2) comprises 0 - 60 mol%, by preference 0 - 50 mol%, preferably 0 - 40 mol%, more preferably 0 - 30 mol%, especially preferably 0 - 20 mol% and particularly preferably 0 - 10 mol% of the entirety of the amount of the free-radical-forming substances (IV) altogether employed in the metered streams (D1) and (D2).

7. The process according to any of claims 1 to 6, wherein the entirety of the compounds (II) and (IV) in the metered stream (D1) is 75 to 100 wt%, preferably 80 to 100 wt%, more preferably 85 to 100 wt%, particularly preferably 90 to 100 wt%, in each case based on the total weight of the metered stream (D1).

8. The process according to any of claims 2 to 7, wherein the metered streams (D1) and (D2) are metered into the reactor predominantly simultaneously, preferably simultaneously.

9. The process according to any of claims 2 to 8, wherein the metering of the metered streams (D1) and (D2) into the reactor is effected substantially continuously, preferably continuously.

10. The process according to any of claims 1 to 9, wherein one, more than one or all of the free-radical-forming substances (IV) conform to formula (V) 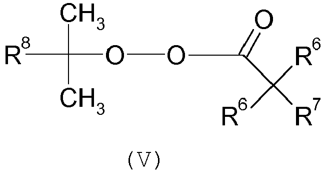 where
R⁶ independently at each occurrence represents hydrogen, (C₁-C₁₀)-alkyl, by preference (C₁-C₆)-alkyl, preferably (C₁-C₄)-alkyl,
R⁷ represents hydrogen or (C₁-C₁₀)-alkyl, by preference hydrogen or (C₁-C₆)-alkyl, preferably hydrogen or (C₁-C₄)-alkyl,
and
R⁸ represents methyl, ethyl, 2,2-dimethylpropyl or phenyl.

11. The process according to any of claims 1 to 10, wherein the reaction is effected at a temperature in the range from 40°C to 120°C, preferably at a temperature in the range from 50°C to 110°C, more preferably at a temperature in the range from 55°C to 100°C and particularly preferably at a temperature in the range from 60°C to 95°C.

12. The process according to any of claims 1 to 11, wherein the molar ratio of the entirety of the employed compound of formula (II) to the entirety of the employed compound of formula (III) is in the range from 8 : 1 to 1 : 1, preferably in the range from 5 : 1 to 2 : 1.

13. The process according to any of claims 1 to 12, wherein
one of the compounds or the compound of formula (II) corresponds to formula (IIa) 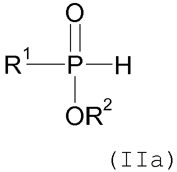 where R¹
represents (C₁-C₆)-alkyl, (C₁-C₆)-haloalkyl, (C₆-C₈)-aryl, (C₆-C₈)-haloaryl, (C₇-C₁₀)-aralkyl, (C₇-C₁₀)-haloaralkyl, (C₅-C₈)-cycloalkyl or (C₅-C₈)-halocycloalkyl and
R² represents (C₁-C₆)-alkyl, (C₁-C₆)-haloalkyl, (C₆-C₈)-aryl, (C₆-C₈)-haloaryl, (C₇-C₁₀)-aralkyl, (C₇-C₁₀)-haloaralkyl, (C₅-C₈)-cycloalkyl or (C₅-C₈)-halocycloalkyl,
and
one of the compounds or the compound of formula (III) corresponds to formula (IIIA)

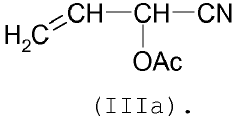

14. A process for producing glufosinate 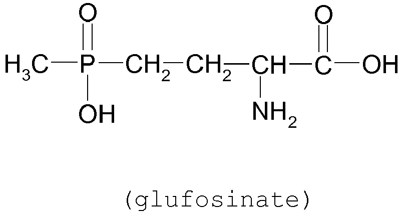 or glufosinate salts, wherein in this process a compound of formula (Ib) is employed 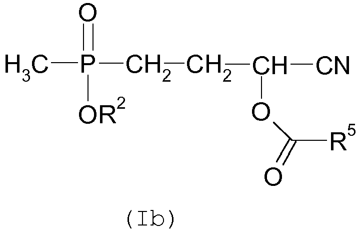
where R² has the meaning recited in claim 1 or claim 13,
R⁵ has the meaning recited in claim 1 or represents methyl and
the production of the compound of formula (Ib) is effected by a process defined in claims 1 to 13.

15. A process for producing glufosinate/glufosinate salts, in particular glufosinate, glufosinate sodium or glufosinate ammonium, comprising the following steps (a) and (b):
a) production of a compound of formula (I)/(Ib) as defined in claim 1, claim 13 or claim 14 and produced by a process defined in claims 1 to 13,
b) use of the compound of formula (I)/(Ib) produced in step (a) for producing glufosinate/glufosinate salts, in particular glufosinate, glufosinate sodium or glufosinate ammonium.

## Revendications

1. Procédé de fabrication d'un composé de formule (I) 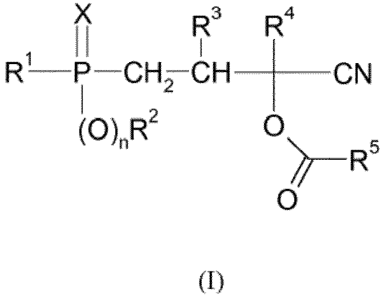 **caractérisé en ce qu'**un composé de formule (II) 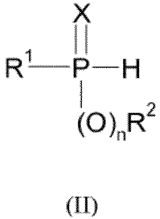 est mis en réaction avec un composé de formule (III) 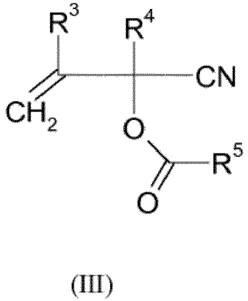 dans lesquelles
R¹ signifie alkyle en (C₁-C₁₂), haloalkyle en (C₁-C₁₂), aryle en (C₆-C₁₀), haloaryle en (C₆-C₁₀), aralkyle en (C₇-C₁₀), haloaralkyle en (C₇-C₁₀), cycloalkyle en (C₄-C₁₀) ou halocycloalkyle en (C₄-C₁₀),
R² signifie alkyle en (C₁-C₁₂), haloalkyle en (C₁-C₁₂), aryle en (C₆-C₁₀), haloaryle en (C₆-C₁₀), aralkyle en (C₇-C₁₀), haloaralkyle en (C₇-C₁₀), cycloalkyle en (C₄-C₁₀) ou halocycloalkyle en (C₄-C₁₀),
R³ et R⁴ signifient chacun indépendamment l'un de l'autre hydrogène, alkyle en (C₁-C₄), phényle ou benzyle,
R⁵ signifie alkyle en (C₁-C₁₂), haloalkyle en (C₁-C₁₂), aryle en (C₆-C₁₀), haloaryle en (C₆-C₁₀), aralkyle en (C₇-C₁₀), haloaralkyle en (C₇-C₁₀), cycloalkyle en (C₄-C₁₀) ou halocycloalkyle en (C₄-C₁₀),
X signifie oxygène ou soufre, et
n représente 0 ou 1,
en présence d'une ou de plusieurs substances formant des radicaux (IV),
un courant d'ajout (D1) qui contient un ou plusieurs composés de formule (II) et une ou plusieurs substances formant des radicaux (IV) étant ajouté dans le réacteur, et le courant d'ajout (D1) contenant 25 à 100 % en moles de la quantité totale de la quantité totale utilisée dans la réaction des substances formant des radicaux (IV).

2. Procédé selon la revendication 1, **caractérisé en ce que** deux courants d'ajout (D1) et (D2) séparés sont ajoutés dans le réacteur, et ces courants d'ajout (D1) et (D2) présentent la composition suivante :
le courant d'ajout (D1) contient un ou plusieurs composés de formule (II) et une ou plusieurs substances formant des radicaux (IV), le courant d'ajout (D1) contenant 25 à 100 % en moles de la quantité totale de la quantité totale utilisée dans la réaction des substances formant des radicaux (IV),
et
le courant d'ajout (D2) contient un ou plusieurs composés de formule (III), ainsi qu'éventuellement un ou plusieurs composés de formule (II) et éventuellement une ou plusieurs substances formant des radicaux (IV).

3. Procédé selon la revendication 1 ou 2, **caractérisé en ce que** le courant d'ajout (D1) contient 30 à 100 % en moles de la quantité totale de la quantité totale utilisée dans la réaction des substances formant des radicaux (IV), de préférence 40 à 100 % en moles, de préférence 50 à 100 % en moles, de manière davantage préférée 60 à 100 % en moles, de manière encore davantage préférée 70 à 100 % en moles, de manière particulièrement préférée 80 à 100 % en moles, de manière notamment préférée 90 à 100 % en moles et de manière préférée entre toutes 95 à 100 % en moles.

4. Procédé selon l'une quelconque des revendications 2 ou 3, **caractérisé en ce que** le courant d'ajout (D1) contient 90 à 100 % en poids, de préférence 95 à 100 % en poids, de manière davantage préférée 100 % en poids, de la quantité totale de la quantité totale utilisée dans les courants d'ajout (D1) et (D2) de composés de formule (II).

5. Procédé selon l'une quelconque des revendications 2 à 4, **caractérisé en ce que**
le courant d'ajout (D2) contient 80 à 100 % en poids, de préférence 90 à 100 % en poids, de préférence 95 à 100 % en poids, de manière notamment préférée 100 % en poids, de la quantité totale de la quantité totale utilisée dans les courants d'ajout (D1) et (D2) de composés de formule (III).

6. Procédé selon l'une quelconque des revendications 2 à 5, **caractérisé en ce que**
le courant d'ajout (D1) contient 40 à 100 % en moles, de préférence 50 à 100 % en moles, de préférence 60 à 100 % en moles, de manière davantage préférée 70 à 100 % en moles, de manière notamment préférée 80 à 100 % en moles, et de manière particulièrement préférée 90 à 100 % en moles, de la quantité totale de la quantité totale utilisée dans les courants d'ajout (D1) et (D2) des substances formant des radicaux (IV),
et/ou
le courant d'ajout (D2) contient 0 à 60 % en moles, de préférence 0 à 50 % en moles, de préférence 0 à 40 % en moles, de manière davantage préférée 0 à 30 % en moles, de manière notamment préférée 0 à 20 % en moles, et de manière particulièrement préférée 0 à 10 % en moles, de la quantité totale de la quantité totale utilisée dans les courants d'ajout (D1) et (D2) des substances formant des radicaux (IV).

7. Procédé selon l'une quelconque des revendications 1 à 6, **caractérisé en ce que** la quantité totale des composés (II) et (IV) dans le courant d'ajout (D1) est de 75 à 100 % en poids, de préférence de 80 à 100 % en poids, de manière davantage préférée de 85 à 100 % en poids, de manière particulièrement préférée de 90 à 100 % en poids, à chaque fois par rapport au poids total du courant d'ajout (D1).

8. Procédé selon l'une quelconque des revendications 2 à 7, **caractérisé en ce que** les courants d'ajout (D1) et (D2) sont ajoutés essentiellement simultanément, de préférence simultanément, dans le réacteur.

9. Procédé selon l'une quelconque des revendications 2 à 8, **caractérisé en ce que** l'ajout des courants d'ajout (D1) et (D2) dans le réacteur a lieu essentiellement en continu, de préférence en continu.

10. Procédé selon l'une quelconque des revendications 1 à 9, **caractérisé en ce qu'**une, plusieurs ou toutes les substances formant des radicaux (IV) correspondent à la formule (V) : 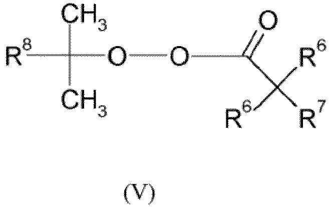 dans laquelle
les R⁶ signifient indépendamment les uns des autres hydrogène, alkyle en (C₁-C₁₀), de préférence alkyle en (C₁-C₆), de préférence alkyle en (C₁-C₄),
R⁷ signifie hydrogène ou alkyle en (C₁-C₁₀), de préférence hydrogène ou alkyle en (C₁-C₆), de préférence hydrogène ou alkyle en (C₁-C₄),
et
R⁸ signifie méthyle, éthyle, 2,2-diméthylpropyle ou phényle.

11. Procédé selon l'une quelconque des revendications 1 à 10, **caractérisé en ce que** la réaction a lieu à une température dans la plage allant de 40 à 120 °C, de préférence à une température dans la plage allant de 50 à 110 °C, de manière davantage préférée à une température dans la plage allant de 55 à 100 °C, et de manière particulièrement préférée à une température dans la plage allant de 60 à 95 °C.

12. Procédé selon l'une quelconque des revendications 1 à 11, **caractérisé en ce que** le rapport molaire entre la quantité totale du composé de formule (II) utilisé et la quantité totale du composé de formule (III) utilisé se situe dans la plage allant de 8:1 à 1:1, de préférence dans la plage allant de 5:1 à 2:1.

13. Procédé selon l'une quelconque des revendications 1 à 12, **caractérisé en ce que**
un des composés ou le composé de formule (II) correspond à la formule (IIa) : 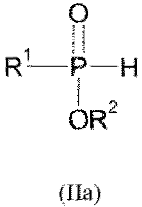 dans laquelle
R¹ signifie alkyle en (C₁-C₆), haloalkyle en (C₁-C₆), aryle en (C₆-C₈), haloaryle en (C₆-C₈), aralkyle en (C₇-C₁₀), haloaralkyle en (C₇-C₁₀), cycloalkyle en (C₅-C₈) ou halocycloalkyle en (C₅-C₈), et
R² signifie alkyle en (C₁-C₆), haloalkyle en (C₁-C₆), aryle en (C₆-C₈), haloaryle en (C₆-C₈), aralkyle en (C₇-C₁₀), haloaralkyle en (C₇-C₁₀), cycloalkyle en (C₅-C₈) ou halocycloalkyle en (C₅-C₈),
et
un des composés ou le composé de formule (III) correspond à la formule (IIIa) :

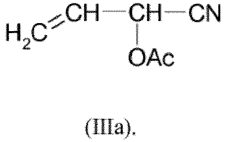

14. Procédé de fabrication de glufosinate 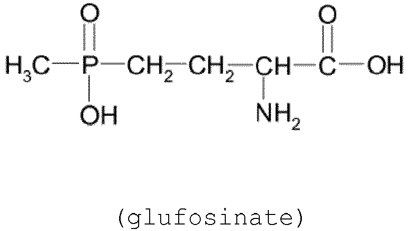 ou de sels de glufosinate, **caractérisé en ce que**, dans ce procédé, un composé de formule (Ib) est utilisé 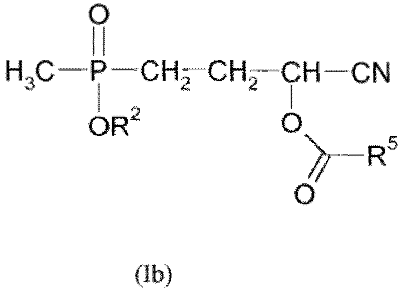
dans laquelle R² a la signification indiquée dans la revendication 1 ou la revendication 13,
R⁵ a la signification indiquée dans la revendication 1 ou signifie méthyle, et
la fabrication du composé de formule (Ib) a lieu par un procédé défini dans les revendications 1 à 13.

15. Procédé de fabrication de glufosinate ou de sels de glufosinate, notamment de glufosinate, de glufosinate-sodium ou de glufosinate-ammonium, comprenant les étapes (a) et (b) suivantes :
(a) la fabrication d'un composé de formule (I) ou (Ib), tel que défini dans la revendication 1, la revendication 13 ou la revendication 14, fabriqué par un procédé défini dans les revendications 1 à 13,
(b) l'utilisation du composé de formule (I) ou (Ib) fabriqué à l'étape (a) pour la fabrication de glufosinate ou de sels de glufosinate, notamment de glufosinate, de glufosinate-sodium ou de glufosinate-ammonium.
